# EUROPÄISCHE PATENTSCHRIFT

(11) **EP 3 056 239 B1**
(45) Veröffentlichungstag und Bekanntmachung des Hinweises auf die Patenterteilung: **28.10.2020**
(21) Anmeldenummer: 16154040.6
(22) Anmeldetag: 03.02.2016
(51) Int. Cl.: A61M 25/00, A61M 39/24, A61M 25/10, A61M 25/01

(54) **BALLONKATHETER**
BALLOON CATHETER
CATHÉTER À BALLONNET

(30) Priorität: 04.02.2015 DE 102015101613
(43) Veröffentlichungstag der Anmeldung: 17.08.2016
(73) Patentinhaber: MTW-Endoskopie W. Haag KG, 46487 Wesel (DE)
(72) Erfinder: Haag, Wolfgang, 46487 Wesel (DE)
(74) Vertreter: Brötz, Helmut

(56) Entgegenhaltungen:
- DE-A1-102012 109 894
- US-A- 3 982 544
- US-A1- 2012 035 588

## Beschreibung

Die vorliegende Erfindung betrifft einen Ballonkatheter umfassend eine Katheterleitung, einen daran angebrachten, dehnbaren Ballon, der einen Balloninnenraum berandet, und eine mit der Katheterleitung verbundene Anschlusseinrichtung, die zumindest einen ersten Anschluss zur Zufuhr von Fluid, insbesondere von Luft, zum Befüllen des Ballons aufweist, wobei sich in der Katheterleitung zumindest ein erster Kanal erstreckt, der an seinem proximalen Längsende mit dem ersten Anschluss in fluidischer Verbindung steht und der an seinem distalen Längsende mit dem Balloninnenraum in fluidischer Verbindung steht, wobei der erste Kanal einen ersten Kanallängenabschnitt und einen sich daran in distaler Richtung mit einem Übergang anschließenden zweiten Kanallängenabschnitt aufweist und wobei die Katheterleitung zumindest entlang dieser Kanallängenabschnitte und entlang des Übergangs aus elastischem Material hergestellt ist, wobei der Ballonkatheter zumindest ein erstes, vorzugsweise als Kugel ausgebildetes, Ventilelement aufweist, dass in den ersten Kanal eingesetzt ist, wobei, vorzugsweise bei jeweils entspannter Wandung der Katheterleitung, die Fläche des Hohlquerschnitts des ersten Kanals in dem zweiten Kanallängenabschnitt kleiner als die Fläche des Hohlquerschnitts des ersten Kanals in dem ersten Kanallängenabschnitt und kleiner als die Fläche des senkrecht zur Längsrichtung der Katheterleitung orientierten Querschnitts des ersten Ventilelements ist, und wobei der Ballonkatheter eine Kraftübertragungseinrichtung aufweist.

Ein Ballonkatheter ist aus der nachveröffentlichten deutschen Patentanmeldung DE 10 2013110 989 der Anmelderin bekannt. Dort befindet sich in dem ersten Kanal der Katheterleitung ein Federrückschlagventil, welches ein Zurückströmen von in den Ballon gepumpter Luft verhindert oder zumindest verzögert. Wenngleich der bekannte Ballonkatheter eine Reihe von Vorteilen aufweist, kann dabei ein rasches Entleeren des Ballons nur durch Abtrennen eines hinteren, das Ventil einschließenden Längenabschnittes der Katheterleitung erreicht werden. Auch besitzt der bekannte Ballonkatheter im Hinblick auf das darin enthaltene Federrückschlagventil einen vergleichsweise aufwändigen Aufbau.

Ein gattungsgemäßer Ballonkatheter ist im Stand der Technik aus US 3,982,544 A bekannt. DE 10 2012 109 894 A1 offenbart einen Ballonkatheter und ein Verfahren zu dessen Verwendung, wobei der Ballonkatheter eine Anschlusseinrichtung und in der Katheterleitung ein Ventil aufweist und wobei vorgeschlagen wird, dass nach einer mittels Fluidzufuhr erreichten Ballonaufweitung die Katheterleitung zwischen dem Ventil und der Anschlusseinrichtung durchtrennt wird. US 2012/0035588 A1 offenbart einen Ballonkatheter zur Zufuhr von therapeutischen Substanzen.

Vor diesem Hintergrund liegt der vorliegenden Erfindung die Aufgabe zugrunde, einen gattungsgemäßen Ballonkatheter vorteilhaft weiterzubilden, so dass insbesondere einzelne oder mehrere der zuvor beschriebenen Nachteile zu vermeiden sind.

Diese Aufgabe wird von der Erfindung gemäß Anspruch 1 in Verbindung mit den Merkmalen gelöst, dass die Kraftübertragungseinrichtung an dem ersten Ventilelement befestigt ist und sich von dem ersten Ventilelement in proximaler Richtung durch den ersten Kanal und weiter durch die Anschlusseinrichtung hindurch bis zu einem Griffstück erstreckt, wobei mittels Bewegung des Griffstückes zu der Anschlusseinrichtung das erste Ventilelement aus dem ersten Kanallängenabschnitt in den zweiten Kanallängenabschnitt unter dortiger elastischer Dehnung der Wandung der Katheterleitung bewegbar ist.

Die Bezugnahme auf einen entspannten Zustand der den ersten Kanal berandenden Wandung der Katheterleitung bezieht sich auf die elastische Eigenschaft ihres Materials. Demzufolge ist die Wandung der Katheterleitung entspannt, wenn sie keiner Verformungsspannung unterliegt. Insbesondere ist damit in Bezug auf einen jeweils betrachteten Abschnitt der Katheterleitung gemeint, dass die dortige Wandung nicht durch im Inneren des ersten Kanals befindliche Ventilelemente aufgeweitet wird. Der Begriff erster Kanal bezeichnet einen sich in der Katheterleitung erstreckenden Hohlraum. Die Fläche des Hohlquerschnitts des ersten Kanals umfasst demzufolge jeweils nur die Fläche des Querschnitts des Hohlraums und nicht die Fläche des Querschnitts der den Hohlraum umgebenden Wandung der Katheterleitung. Daher könnte man hier wie im Folgenden von Querschnitt des ersten Kanals anstelle von Hohlquerschnitt des ersten Kanals und demzufolge von Fläche des Querschnitts des ersten Kanals anstelle von Fläche des Hohlquerschnitts des ersten Kanals sprechen. Der angesprochene Hohlquerschnitt bzw. Querschnitt ist insbesondere senkrecht zu der örtlichen Längsrichtung der Katheterleitung orientiert. Bei dem angesprochenen Querschnitt des ersten Ventilelements handelt es sich um den größten senkrecht zur Längsrichtung der Katheterleitung orientierten Querschnitt. Wenn das Ventilelement als Kugel ausgebildet ist, wird der Querschnitt und seine Fläche von dem Durchmesser bestimmt. Bei der Kraftübertragungseinrichtung handelt es sich bevorzugt, wie noch nachfolgend beschrieben, um eine Drahtverbindung. In Bezug auf den Ballonkatheter, die Katheterleitung und die weiteren Komponenten werden die Begriffe proximal und distal in der für Ballonkatheter üblichen Bedeutung verwendet. Dabei bezeichnet der Begriff proximal die einem Anwender bzw. die der Anschlusseinrichtung zugewandte Seite bzw. einen jeweiligen einem Anwender (bspw. Operateur) näheren Ort einer betrachteten Komponente des Ballonkatheters, während der Begriff distal die von dem Anwender entferntere Seite bzw. einen von dem Anwender entfernteren Ort bezeichnet. Entsprechend wird unter proximaler Richtung eine zu dem Anwender hin weisende Richtung und unter distaler Richtung eine von dem Anwender weg weisende Richtung verstanden. Die Erfindung ermöglicht, dass das Ventilelement mittels der Kraftübertragungseinrichtung, die man auch als Ventilschieber bezeichnen könnte, mehrfach in dem ersten Kanal der Katheterleitung in deren Längsrichtung hin- und herbewegt werden kann. Das im Zusammenwirken des Ventilelements mit den beiden Kanallängenabschnitten und ihrem dazwischen liegenden Übergang gebildete Ventil in dem ersten Kanal kann dadurch mehrfach gezielt geschlossen und wieder geöffnet werden. Im Vergleich zu dem bekannten Ballonkatheter ist also zum Entlüften des Ballons nicht erforderlich, dass das Ventil abgetrennt wird. Der Hohlquerschnitt in dem ersten Kanallängenabschnitt ist so groß bemessen, dass Fluid, vorzugsweise Luft, zum Befüllen des Ballons, in distaler Richtung an dem ersten Ventilelement vorbeiströmen kann. Dazu kann die Fläche des Hohlquerschnitts des ersten Kanals in dem ersten Kanallängenabschnitt, insbesondere bezüglich des Flächeninhalts, so groß gewählt sein, dass schon bei darin wirkendem Umgebungsdruck ein Spalt zwischen dem ersten Ventilelement und der den ersten Kanal berandenden Wandung der Katheterleitung resultiert. Alternativ könnte die Fläche des Hohlquerschnitts des ersten Kanals im ersten Kanallängenabschnitt, insbesondere bezüglich des Flächeninhalts, flächenmäßig etwa gleich groß wie oder etwas kleiner als die Fläche des maximalen senkrecht zu der Längsrichtung der Katheterleitung orientierten Querschnittes des ersten Ventilelements gewählt sein, so dass ein Spalt dazwischen erst bei Erreichen eines gewissen über dem Umgebungsdruck liegenden Druckes in dem ersten Kanal gebildet wird. Beim Eintritt des Ventilelementes in den zweiten Kanallängenabschnitt oder in den Übergang zwischen erstem und zweitem Kanallängenabschnitt dichtet es den ersten Kanal ab. Das Ventilelement bildet somit mit den beiden Kanallängenabschnitten und dem dazwischen liegenden Übergang des ersten Kanals ein Ventil, das sich mittels der Kraftübertragungseinrichtung (Ventilschieber) bei Bedarf mehrfach und schnell öffnen und schließen lässt. Das erste Ventilelement ist an seiner distalen Seite vorzugsweise in distaler Richtung verjüngt, bspw. konisch oder gerundet. Dies begünstigt die Dichtwirkung bereits in dem Übergang. Vorzugsweise kann die Kraftübertragungseinrichtung eine, bspw. aus mehreren Drahtabschnitten zusammengesetzte, Drahtverbindung sein. Bevorzugt ist die Drahtverbindung zug- und schubsteif in Bezug auf die im Betrieb auftretenden Kräfte ausgeführt. Vorzugsweise ist der Hohlquerschnitt im ersten Kanallängenabschnitt des ersten Kanals geringfügig größer als der Querschnitt der Drahtverbindung, so dass Fluid hindurch strömen kann und trotzdem eine zumindest im Wesentlichen konzentrische Führung der Drahtverbindung in dem ersten Kanal resultiert. Dies gilt entsprechend für einen Durchlass für die Kraftübertragungseinrichtung und für Fluid durch die Anschlusseinrichtung. Wenn das erste Ventilelement in den zweiten Kanallängenabschnitt des ersten Kanals eintritt, drückt sich die Oberfläche des Ventilelements fest gegen die Oberfläche der elastischen Wandung des ersten Kanals, wodurch eine zuverlässige Dichtwirkung entsteht. Zufolge der genannten Querschnittsverhältnisse bewirkt das Eindringen des Ventilelements in den zweiten Kanallängenabschnitt eine elastische Aufweitung der berandenden Wandung der Katheterleitung. Die Dehnungsspannungen bewirken eine feste, für die Dichtung ausreichende Anlage der Wandung an dem Ventilelement. Die Kraftübertragungseinrichtung ist ausreichend steif gestaltet, um in Längsrichtung der Katheterleitung eine Druckkraft auf das erste Ventilelement auszuüben, die ausreicht, um das erste Ventilelement in den zweiten Kanallängenabschnitt hineinzudrücken und dabei die Wandung der Katheterleitung im zweiten Kanallängenabschnitt elastisch aufzuweiten. Wird das Ventilelement mittels der Kraftübertragungseinrichtung wieder in proximaler Richtung aus dem zweiten Kanallängenabschnitt zurückgezogen, verformt sich dort die Wandung der Katheterleitung elastisch zurück.

Es bestehen zahlreiche Möglichkeiten zur bevorzugten Ausgestaltung. So besteht die Möglichkeit, dass die Fläche des Hohlquerschnitts des ersten Kanals an jeder Stelle in dem zweiten Kanallängenabschnitt kleiner als die Fläche des Hohlquerschnitts des ersten Kanals an jeder Stelle in dem ersten Kanallängenabschnitt und kleiner als die Fläche des größten senkrecht zur Längsrichtung der Katheterleitung orientierten Querschnitts des ersten Ventilelements ist. Der Hohlquerschnitt kann bspw. rund (insbesondere kreisrund oder oval) oder mehreckig berandet sein. Bevorzugt ist, dass, insbesondere bei jeweils entspannter Wandung der Katheterleitung, der Hohlquerschnitt des ersten Kanals in dem ersten Kanallängenabschnitt einheitlich oder im Wesentlichen einheitlich ist und der hiervon flächenmäßig abweichende Hohlquerschnitt des ersten Kanals in dem zweiten Kanallängenabschnitt darin ebenfalls einheitlich oder im Wesentlichen einheitlich ist. Dies kann sich auf die Form und die Größe des Hohlquerschnittes beziehen. Von im Wesentlichen einheitlich ist bspw. zu sprechen, wenn etwaige Größenunterschiede nur im einstelligen Prozentbereich liegen. Ein innerhalb der jeweiligen Kanallängenabschnitte einheitlicher oder im Wesentlichen einheitlicher Hohlquerschnitt ist aber nicht zwingend, sondern die Erfindung schließt auch die Möglichkeit ein, dass der Hohlquerschnitt innerhalb bzw. entlang eines jeweiligen Kanallängenabschnittes variiert.

Es besteht die Möglichkeit, dass, insbesondere bei jeweils entspannter Wandung der Katheterleitung, der Hohlquerschnitt des ersten Kanals in dem ersten Kanallängenabschnitt einheitlich oder im Wesentlichen einheitlich ist und der hiervon flächenmäßig abweichende Hohlquerschnitt des ersten Kanals in dem zweiten Kanallängenabschnitt darin ebenfalls einheitlich oder im Wesentlichen einheitlich ist. Dabei ist bevorzugt, dass bei Betrachtung eines Querschnitts der Katheterleitung die besagte Fläche des Hohlquerschnittes des ersten Kanals, also die im Querschnitt den entsprechenden Hohlraum repräsentierende Fläche, schon im entspannten Zustand der Wandung größer als die angesprochene Querschnittsfläche des Ventilelementes ist. Bevorzugt ist, dass, insbesondere bei jeweils entspannter Wandung der Katheterleitung der erste Kanal in dem ersten Kanallängenabschnitt einheitlich einen kreisförmigen Hohlquerschnitt mit einem in dem ersten Kanallängenabschnitt einheitlichen ersten Kanaldurchmesser und in dem zweiten Kanallängenabschnitt einheitlich einen kreisförmigen Hohlquerschnitt mit einem in dem zweiten Kanallängenabschnitt einheitlichen zweiten Kanaldurchmesser besitzt, wobei der zweite Kanaldurchmesser kleiner als der erste Kanaldurchmesser und kleiner als der Durchmesser des insbesondere als Kugel oder Halbkugel ausgebildeten ersten Ventilelements ist.

Es besteht die Möglichkeit, dass, insbesondere bei jeweils entspannter Wandung der Katheterleitung, im ersten Kanallängenabschnitt der erste Kanaldurchmesser größer als oder gleich groß wie oder kleiner als der Durchmesser des als Kugel oder Halbkugel ausgebildeten ersten Ventilelements ist.

Als zweckmäßig wird angesehen, dass sich in dem ersten Kanal in dem Übergang zwischen dem ersten Kanallängenabschnitt und dem zweiten Kanallängenabschnitt der Hohlquerschnitt des ersten Kanals in distaler Richtung kontinuierlich, insbesondere konisch, verjüngt. Dies begünstigt ein zuverlässiges Öffnen und Schließen des gebildeten Ventils.

Bei einem bevorzugten Ausführungsbeispiel ist vorgesehen, dass an der Kraftübertragungseinrichtung ein erster Anschlag befestigt oder ausgebildet ist und dass an der Anschlusseinrichtung ein erster Gegenanschlag befestigt oder ausgebildet ist und dass das Griffstück zu der Anschlusseinrichtung bis zu einer distalen Endposition vorschiebbar ist, bei deren Erreichen der erste Anschlag, die Verschiebung formschlüssig begrenzend, gegen den ersten Gegenanschlag tritt und sich das erste Ventilelement in dem zweiten Kanallängenabschnitt befindet. Alternativ oder kombinativ besteht die Möglichkeit, dass an der Kraftübertragungseinrichtung ein zweiter Anschlag befestigt oder ausgebildet ist und an der Anschlusseinrichtung ein zweiter Gegenanschlag befestigt oder ausgebildet ist und dass das Griffstück von der Anschlusseinrichtung bis zu einer proximalen Endposition wegziehbar ist, bei deren Erreichen der zweite Anschlag, die Ziehbewegung formschlüssig begrenzend, gegen den zweiten Gegenanschlag tritt und sich das erste Ventilelement in dem ersten Kanallängenabschnitt befindet. Dies vereinfacht und begünstigt jeweils eine zuverlässige und reproduzierbare Verlagerung des Ventilelements bzw. der Ventilelemente in eine gewünschte Position in dem ersten oder in dem zweiten Kanallängenabschnitt, wodurch eine besonders zuverlässige Ventilfunktion resultiert.

Bevorzugt ist, dass der Ballonkatheter ein zweites, insbesondere zylinderförmiges, Ventilelement aufweist, das an das erste Ventilelement an dessen proximaler Seite angrenzt, wobei insbesondere vorgesehen ist, dass der Durchmesser des zweiten Ventilelements dem Durchmesser des ersten Ventilelements entspricht, wobei, wenn sich das Griffstück in der distalen Endposition befindet, sich das zweite Ventilelement ganz oder teilweise in dem zweiten Kanallängenabschnitt des ersten Kanals befindet. Mittels des zweiten Ventilelements kann die Dichtwirkung noch weiter verbessert werden. Bevorzugt ist, dass das proximale Längsende des zweiten Kanallängenabschnittes von der Anschlusseinrichtung in distaler Richtung beabstandet ist. Als zweckmäßig wird angesehen, dass die Außenoberfläche der Katheterleitung zumindest entlang dem ersten und zweiten Kanallängenabschnitt und dem Übergang aus Kunststoff, wie bspw. Polytetrafluorethylen (PTFE), oder aus Gummi besteht. Es besteht die Möglichkeit, dass die Katheterleitung in ihrem dem ersten Kanallängenabschnitt entsprechenden Längenbereich dicker als in ihrem dem zweiten Kanallängenabschnitt entsprechenden Längenbereich ist. Als zweckmäßig wird angesehen, dass die Katheterleitung, zumindest in einem den ersten Kanallängenabschnitt, den Übergang und den zweiten Kanallängenabschnitt einschließenden Längenteilabschnitt, oder insgesamt einstückig ausgebildet ist. Zum Beispiel besteht die Möglichkeit, dass der Übergang mit dem sich verjüngenden Hohlquerschnitt hergestellt wird, indem die Katheterleitung in erwärmtem Zustand bis zu einer bestimmten Position durch ein hülsenartiges, sich verjüngendes Werkzeug gezogen wird. Die Position des Überganges wird dadurch bestimmt, wie weit die Katheterleitung durch das Werkzeug gezogen wird. Der erste Kanallängenabschnitt und der zweite Kanallängenabschnitt können, abhängig von den Anforderungen, in ihrer jeweiligen Länge gewählt werden. Bevorzugt ist, dass sich der erste Kanallängenabschnitt von dem Übergang in proximaler Richtung bis zu dem proximalen Längsende der Katheterleitung erstreckt. Bevorzugt ist, dass sich der zweite Kanallängenabschnitt von dem Übergang in distaler Richtung bis zu einem Verschluss des ersten Kanals erstreckt, der bezüglich eines Durchlasses von dem ersten Kanal zu dem Balloninneren distal angeordnet ist. Bevorzugt ist, dass der Ballonkatheter eine Knickschutzhülse aufweist, die an der Anschlusseinrichtung angebracht ist und die von dort lose einen Längenteilabschnitt der Katheterleitung, der sich von der Anschlusseinrichtung in distaler Richtung bis in den zweiten Kanallängenabschnitt erstreckt, ummantelt, und dass an der Knickschutzhülse eine von außen sichtbare erste Markierung, insbesondere ein Ring in einer ersten Farbe, angebracht ist, wobei die erste Markierung von dem distalen Längsende des ersten Kanallängenabschnitts in proximaler Richtung beabstandet ist. Die erste Markierung kann eine bevorzugte Position angeben, wo der Ballon unter Beibehaltung eines Befüllungszustandes des Ballons, bspw. zum Austausch von auf die Katheterleitung aufgeschobenen Endoskopen, durchtrennt werden kann. Alternativ oder kombinativ besteht die Möglichkeit, dass an der Katheterleitung eine zweite Markierung, insbesondere ein Ring in einer zweiten Farbe, angebracht ist, die, wenn die Katheterleitung freiliegt, von außen sichtbar ist, wobei die zweite Markierung von dem proximalen Längsende des zweiten Kanallängenabschnitts und von dem ersten Ventilelement, auch wenn sich das Griffstück in seiner distalen Endposition befindet, in distaler Richtung beabstandet ist und die insbesondere an dem von der Knickschutzhülse lose ummantelten Längenteilabschnitt der Katheterleitung angeordnet ist. Die zweite Markierung kann eine bevorzugte Position der Katheterleitung angeben, an der diese zu durchschneiden ist, um den Ballon auch noch nach dem Abtrennen der Anschlusseinrichtung zuverlässig entlüften zu können. Wenn, was bevorzugt ist, die Knickschutzhülse blickdicht ist, wird die zweite Markierung zunächst von der Knickschutzhülse verdeckt, was die Funktionssicherheit des Ballonkatheters verbessert.

Bevorzugt ist, dass eine Befüllungseinrichtung vorhanden ist, die eine Hohlnadel und ein daran anschließendes Gehäuse umfasst, wobei an der Befüllungseinrichtung zumindest ein Anschluss zur Zufuhr von Fluid zum Befüllen des Ballons, insbesondere ein Anschluss zur lösbaren Befestigung einer Spritze, ausgebildet ist, dass der Anschluss und die Hohlnadel mittels eines sich in dem Gehäuse erstreckenden Fluiddurchlasses miteinander in fluidischer Verbindung stehen und dass der Außenquerschnitt der Hohlnadel geringfügig größer als der Hohlquerschnitt des ersten Kanals in dem zweiten Kanallängenabschnitt ist. Dies ermöglicht ein erneutes Befüllen des Ballons auch nach dem Durchtrennen der Katheterleitung an der besagten zweiten Markierung. Als zweckmäßig wird angesehen, dass die Anschlusseinrichtung einen dritten Anschluss aufweist und dass die Katheterleitung einen zweiten Kanal aufweist, der den dritten Anschluss mit einer Öffnung der Katheterleitung an ihrem distalen Ende verbindet. Der Begriff zweiter Kanal bezeichnet einen weiteren sich in der Katheterleitung erstreckenden Hohlraum. Der zweite Kanal kann bspw. zum Hindurchführen eines Führungsdrahtes verwendet werden. Auch kann der zweite Kanal zum Hindurchleiten von Kontrastmittel dienen.

Die Erfindung wird nachfolgend mit Bezug auf die beigefügten Figuren, welche bevorzugte Ausführungsbeispiele zeigen, weiter beschrieben. Darin zeigt:
- Fig. 1: einen erfindungsgemäßen Ballonkatheter gemäß einem ersten bevorzugten Ausführungsbeispiel in einer durch zwei Aufbrüche verkürzten Seitenansicht, vor Beginn der Fluidzufuhr in den Ballon;
- Fig. 1a: Detail Ia aus Fig. 1 in einer geschnittenen Vergrößerung;
- Fig. 1b: Detail Ib aus Fig. 1 in einer geschnittenen Vergrößerung;
- Fig. 2: den Ballonkatheter aus Fig. 1, jedoch nach Zufuhr von Fluid mit aufgepumptem Ballon;
- Fig. 2a: Detail IIa aus Fig. 2 in einer geschnittenen Vergrößerung;
- Fig. 2b: Detail IIb aus Fig. 2 in einer geschnittenen Vergrößerung;
- Fig. 3: den Ballonkatheter gemäß Fig. 1 und 2, jedoch nach Zufuhr von Fluid und nach Schließen des zum Fluidtransport dienenden ersten Kanals der Katheterleitung;
- Fig. 3a: Detail IIIa aus Fig. 3 in einer geschnittenen Vergrößerung;
- Fig. 3b: Detail IIIb aus Fig. 3 in einer geschnittenen Vergrößerung;
- Fig. 4: in einer Seitenansicht eine aus dem in den Figuren 1 bis 3 gezeigten Ballonkatheter entnommene Anordnung, umfassend eine Kraftübertragungseinrichtung und daran distal angebrachte erste und zweite Ventilelemente;
- Fig. 4a: Detail IVa aus Fig. 4 in Vergrößerung und teilweise geschnitten;
- Fig. 5: die Anschlusseinrichtung des in den Figuren 1 bis 3 gezeigten Ballonkatheters, demgegenüber in einer vergrößerten Schnittansicht;
- Fig. 6: einen Querschnitt durch die Katheterleitung in dem ersten Kanallängenabschnitt ohne darin befindliche Kraftübertragungseinrichtung, in entspanntem Zustand der Wandung;
- Fig. 7: einen Querschnitt der Katheterleitung in dem zweiten Kanallängenabschnitt, ohne darin befindliche Kraftübertragungseinrichtung, in entspanntem Zustand der Wandung;
- Fig. 8: den Ballonkatheter gemäß Fig. 1 bis 7 in Verbindung mit einem Führungsdraht und mit einem ersten Endoskop in einem exemplarischen Gebrauchszustand;
- Fig. 9: den Ballonkatheter gemäß Fig. 1 bis 7 und das erste Endoskop, in einem späteren exemplarischen Gebrauchszustand;
- Fig. 10: den Ballonkatheter gemäß Fig. 1 bis 7 und ein zweites Endoskop, in einem noch späteren exemplarischen Gebrauchszustand;
- Fig. 11: den Ballonkatheter gemäß Fig. 1 bis 7 und das zweite Endoskop, in einem noch späteren exemplarischen Gebrauchszustand;
- Fig. 12: in einer Seitenansicht eine Befüllungseinrichtung mit darin eingesetztem Mandrin, gemäß einem bevorzugten Ausführungsbeispiel, zur Verwendung mit einem erfindungsgemäßen Ballonkatheter;
- Fig. 13: in einem Längsschnitt die in Fig. 12 gezeigte Befüllungseinrichtung mit eingesetztem Mandrin;
- Fig. 14: in einem Längsschnitt die Befüllungseinrichtung gemäß den Fig. 12, 13, wobei der Mandrin entnommen und eine Spritze zur Fluidzufuhr angeschlossen wurde;
- Fig. 15: ausgehend von Fig. 11 das nach dem Durchtrennen der Katheterleitung gebildete proximale Längsende der noch mit dem Ballon verbundenen Katheterleitung beim Anschließen der Befüllungseinrichtung und
- Fig. 16: die in Fig. 11 gezeigte Anordnung, jedoch mit daran angeschlossener Befüllungseinrichtung und nach dem erneuten Befüllen des Ballons mit Fluid.

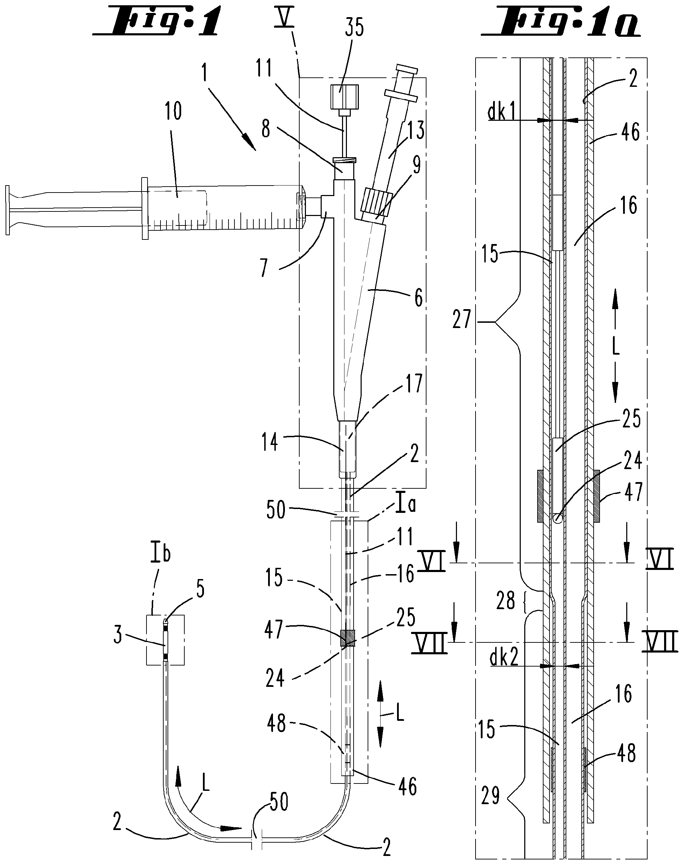

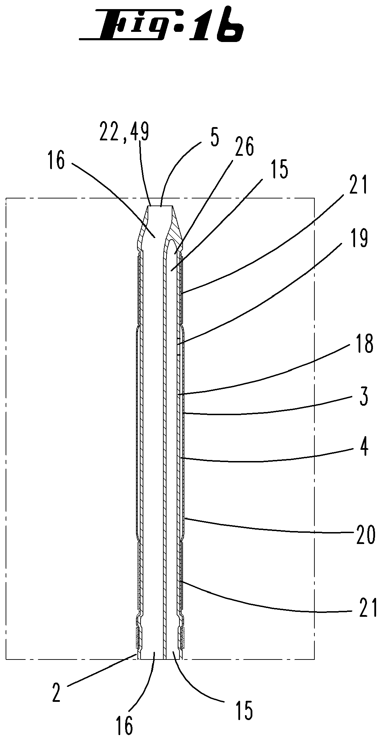

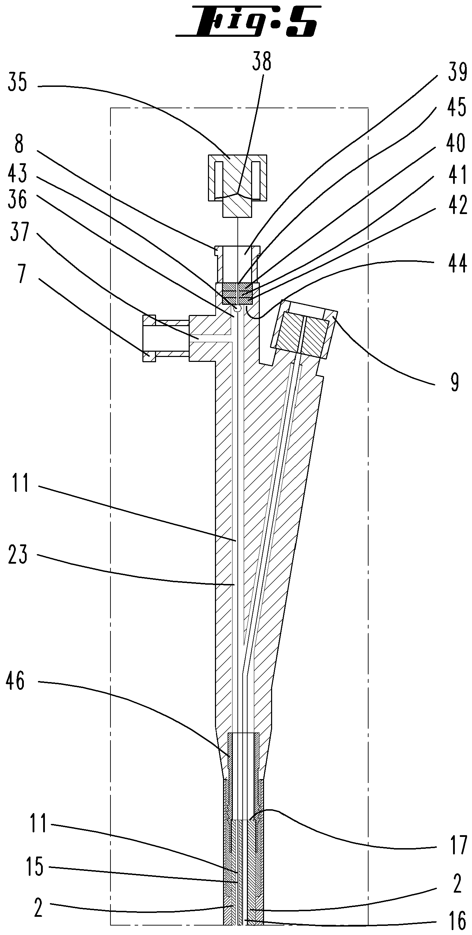

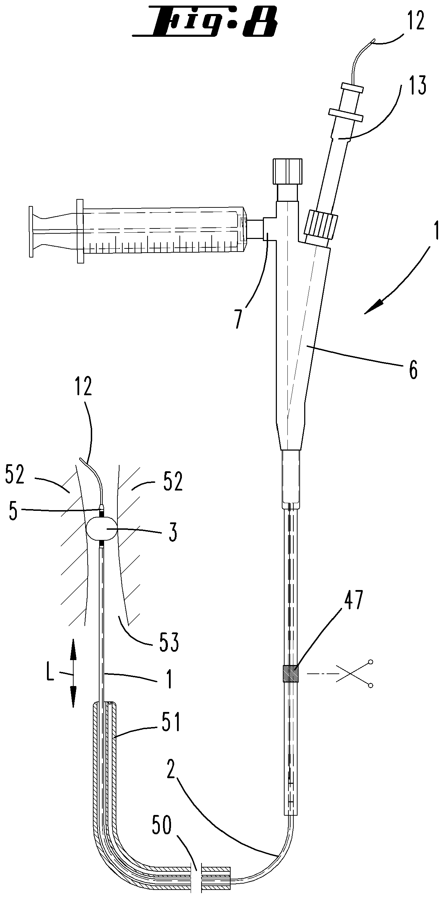

Mit Bezug auf die Figuren 1 bis 7 wird ein erfindungsgemäßer Ballonkatheter 1 gemäß einem bevorzugten Ausführungsbeispiel vorgestellt. Dieser umfasst eine Katheterleitung 2, einen dehnbaren Ballon 3, der einen Balloninnenraum 4 berandet und an der Katheterleitung 2 in der Nähe ihres distalen Längsendes 5 angebracht ist. Des Weiteren umfasst der Ballonkatheter 1 eine Anschlusseinrichtung 6, die an ihrem distalen Längsende fest mit der Katheterleitung 2 verbunden ist. Im Bereich ihres proximalen Endes weist die Anschlusseinrichtung 6 seitlich einen ersten Anschluss 7, nach proximal weisend einen zweiten Anschluss 8 und einen dritten Anschluss 9 auf. Der Anschluss 7 dient zur Zufuhr eines unter Druck stehenden Fluids, bspw. von Luft, um den Ballon 3 aufzupumpen. Zu diesem Zweck ist in dem Beispiel eine Spritze 10 an den Anschluss 7 mittels einer Schraub- oder Steckverbindung angeschlossen. Der zweite Anschluss 8 dient zum Hindurchführen einer nachfolgend noch näher beschriebenen Kraftübertragungseinrichtung 11, die man auch als Ventilschieber bezeichnen könnte. Der Anschluss 9 kann zum Hindurchführen eines Führungsdrahtes 12 (vgl. Figur 8) dienen. Zur Durchleitung von Kontrastmittel kann ein an sich bekannter Kontrastmittelansatz 13 angeschlossen werden. Die Anschlusseinrichtung 6 wird später näher mit Bezug auf Figur 5 beschrieben. Die Katheterleitung 2 ist an ihrem proximalen Längsende 17 mittels einer in Figur 1 nicht näher gezeigten Verbindung 14 fest mit dem distalen Ende der Anschlusseinrichtung 6 verbunden. Dazu ist die Katheterleitung 2 an ihrem proximalen Ende fest in eine Metallhülse eingesetzt, die ihrerseits fest in einer distalen Bohrung der Anschlusseinrichtung 6 montiert ist. Ausgehend von dem proximalen Längsende 17 der Katheterleitung 2 erstrecken sich in der Katheterleitung 2 in deren Längsrichtung L ein hohler erster Kanal 15 und parallel dazu ein hohler zweiter, etwas dickerer Kanal 16. Wie die Figuren 1b, 2b und 3b veranschaulichen, erstreckt sich der erste Kanal 15 bis in den Bereich des distalen Längsendes 5 der Katheterleitung 2, wo er stirnseitig verschlossen ist. In der zur Außenseite der Katheterleitung 2 weisenden äußeren Wandung 18 des ersten Kanals 15 ist eine Durchgangsbohrung 19 ausgebildet, mittels welcher der erste Kanal 15 in fluidischer Verbindung mit dem Balloninnenraum 4 steht. In dem Beispiel ist die Ballonhülle 20 aus einem Schlauchstück aus Latex gebildet, dessen beiden Längsenden mittels je einer Drahtwicklung 21 von außen dichtend gegen den Katheterschlauch 2 angepasst werden. Der zweite Kanal 16 erstreckt sich von dem proximalen Längsende 17 durchgehend bis zu einer stirnseitigen Öffnung 22 am distalen Längsende 5 der Katheterleitung 2.

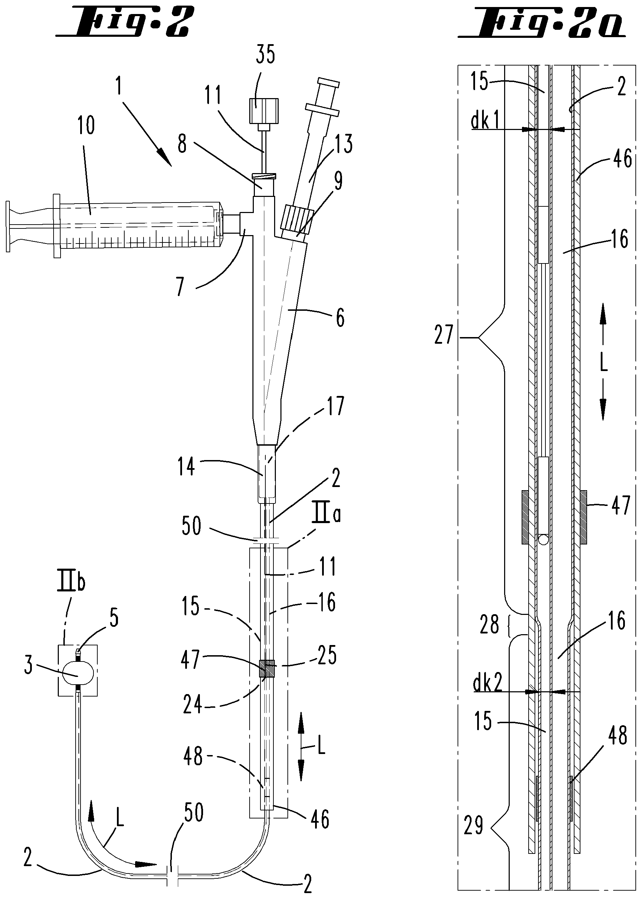

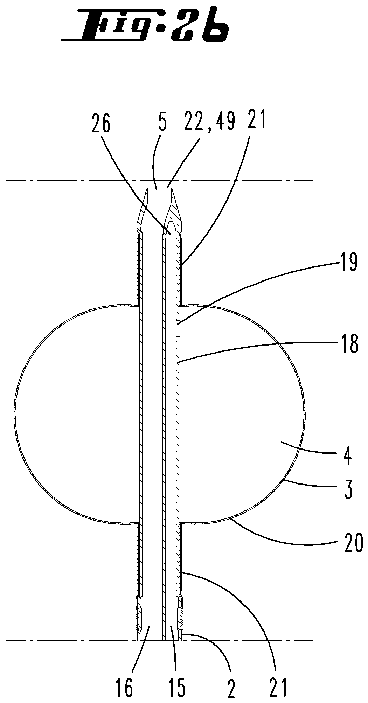

Figur 2 zeigt, dass in der Anschlusseinrichtung 6 der erste Anschluss 7 mittels eines Fluiddurchlasses 23 in fluidischer Verbindung mit dem ersten Kanal 15 steht. Das distale Längsende 26 des ersten Kanals 15 steht in fluidischer Verbindung mit dem Balloninnenraum 4. Zwischen dem proximalen Längsende 17 und dem distalen Längsende 26 sind in den ersten Kanal 15 in dem Beispiel ein erstes Ventilelement 24 und ein zweites Ventilelement 25, jeweils in Längsrichtung L der Katheterleitung 2 verschieblich, eingesetzt. Wie die Figuren 1a, 2a und 3a in Vergrößerung zeigen, weist der erste Kanal 15 einen ersten Kanallängenabschnitt 27 und einen sich daran in distaler Richtung mit einem Übergang 28 anschließenden zweiten Kanallängenabschnitt 29 auf. In den Figuren 1a, 2a und 3a sind der erste Kanallängenabschnitt 27 und der zweite Kanallängenabschnitt 29 nur abschnittsweise sichtbar. In dem Beispiel (d. h. nicht notwendig) erstreckt sich der erste Kanallängenabschnitt 27 von dem Übergang 28 in proximaler Richtung bis zu dem proximalen Längsende 17 der Katheterleitung 2 und erstreckt sich der zweite Kanallängenabschnitt 29 von dem Übergang 28 in distaler Richtung bis zu dem distalen Längsende 26 des ersten Kanals 15. Alternativ wäre aber möglich, dass der von dem Übergang 28 ausgehende erste Kanallängenabschnitt 27 eine geringere Länge besitzt und/oder dass der von dem Übergang 28 ausgehende zweite Kanallängenabschnitt 29 eine kürzere Länge besitzt. Das proximale Längsende 17 der Katheterleitung 2 ist zugleich das proximale Längsende 17 des ersten Kanals 15.

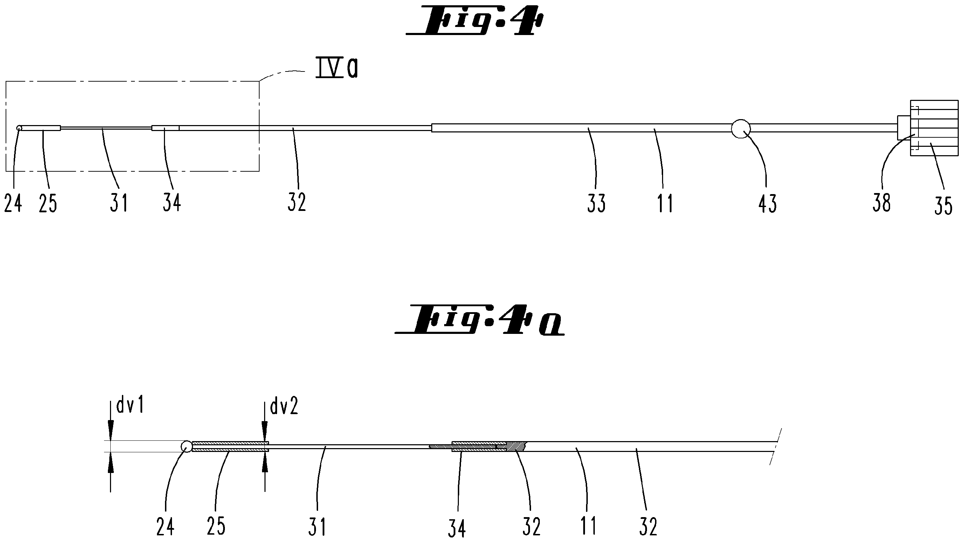

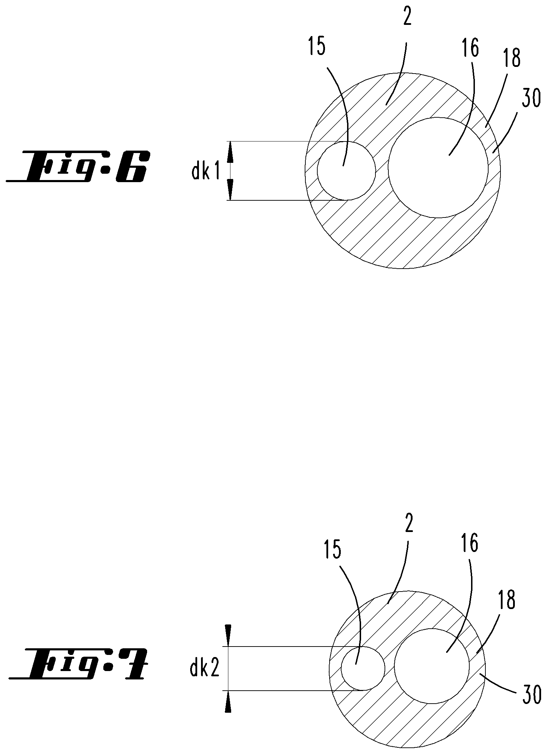

Der Ballonkatheter 2 weist ein erstes Ventilelement 24 auf, das in dem Beispiel als Kugel ausgebildet ist. Bei dem Ausführungsbeispiel ist zusätzlich ein zweites Ventilelement 25 vorgesehen, bei welchem es sich um eine zylindrische Hülse handelt. Die Ventilelemente 24, 25 sind jeweils an der Kraftübertragungseinrichtung 11 befestigt und mittels dieser in Längsrichtung L der Katheterleitung 2 in dem ersten Kanal 15 verschieblich. Der Durchmesser des ersten Ventilelementes 24, d. h. in dem Beispiel der Kugel, ist in Fig. 4a mit dv1 bezeichnet. Der äußere Durchmesser des zweiten Ventilelements 25, d. h. in dem Beispiel der Hülse, ist dort mit dv2 bezeichnet. In dem Beispiel sind die Durchmesser dv1 und dv2 gleich groß gewählt. In dem Beispiel ist die Katheterleitung 2 einstückig aus elastischem Kunststoff hergestellt. Wenn die somit elastisch verformbare, den ersten Kanal 15 umgebende Wandung 30 der Katheterleitung 2 elastisch entspannt ist, besitzt in dem Beispiel der erste Kanal 15 in dem ersten Kanallängenabschnitt 27 einen kreisrunden Querschnitt mit einem in dem ersten Kanallängenabschnitt 27 einheitlichen Kanaldurchmesser dk1 (vgl. Figur 6) und der erste Kanal 15 besitzt in dem zweiten Kanallängenabschnitt 29 (vgl. Figur 7) einen kreisrunden Querschnitt mit einem in dem zweiten Kanallängenabschnitt 29 einheitlichen zweiten Kanaldurchmesser dk2, welcher kleiner als der Durchmesser dk1 ist. Werden jeweils Längenbereiche mit entspannter Wandung 30 betrachtet, ist im gezeigten Ausführungsbeispiel der erste Kanaldurchmesser dk1 größer als die sich entsprechenden Durchmesser dv1 und dv2. Andererseits ist im entspannten Zustand der Durchmesser dk2 kleiner als die Durchmesser dv1 und dv2, wobei dieser Durchmesserunterschied so gewählt ist, dass sich der Durchmesser dk2 beim Eindringen der Ventilelemente 24, 25 auf deren Durchmesser dv1 bzw. dv2 aufweiten lässt. In dem im Beispiel nur vergleichsweise kurzen Übergang 28 nimmt der Durchmesser von dem Durchmesser dk1 zu dem Durchmesser dk2 kontinuierlich ab. Wenn sich die Ventilelemente 24, 25 in dem ersten Kanallängenabschnitt 27 befinden, verbleibt zwischen ihnen und der Wandung 30 der Katheterleitung 2 im Querschnitt ein Spalt, so dass Fluid (in dem Beispiel Luft) in dem ersten Kanal 15 an den Ventilelementen 24, 25 vorbeiströmen kann. Werden andererseits die Ventilelemente 24, 25 aus dem ersten Kanallängenabschnitt 27 in distaler Richtung bewegt, zumindest bis sich das distal vordere Ventilelement 24 in dem Übergang 28 gegen die Wandung 30 andrückt, wird der erste Kanal 15 abgedichtet. Diese Abdichtung kann verstärkt werden, indem das erste Ventilelement 24 und zumindest ein Längenabschnitt des zweiten Ventilelementes 25 in den zweiten Kanallängenabschnitt 29 vorgeschoben werden. Zufolge dieser Abdichtung kann durch den Anschluss 7 zugeführtes, unter einem Druck stehendes Fluid (in dem Beispiel Luft) in dem ersten Kanal 15 nicht an den Ventilelementen 24, 25 in distaler Richtung vorbeiströmen. Die Ventilelemente 24, 25 bilden insofern mit den Kanallängenabschnitten 27, 29 und dem Übergang 28 ein Ventil in dem ersten Kanal 15 aus, das durch Hin- und Herbewegen der Ventilelemente 24, 25 wahlweise geöffnet oder geschlossen werden kann. Ist das Ventil geöffnet, ist der Anschluss 7 mit dem Balloninnenraum 4 fluidisch verbunden, so dass der Ballon 3 mittels durch den Anschluss 7 zugeführter Druckluft aufgepumpt werden kann. Wird das Ventil dann geschlossen, wird dadurch die fluidische Verbindung zwischen dem Anschluss 7 und dem Balloninnenraum 4 unterbrochen, und in dem Ballon 3 enthaltene Druckluft wird an einem Zurückströmen zu dem Anschluss 7 gehindert (d. h. der Ballon 3 bleibt aufgepumpt). Wird danach das Ventil erneut geöffnet, kann die Druckluft aus dem Ballon 3 durch den Anschluss 7 entweichen, so dass sich der elastische Ballon 3 wieder kontrahiert. Aus den beschriebenen geometrischen Beziehungen folgt, dass die Fläche des Hohlquerschnitts des ersten Kanals 15 in dem zweiten Kanallängenabschnitt 29 kleiner als der Hohlquerschnitt des ersten Kanals 15 in dem ersten Kanallängenabschnitt 27 und kleiner als die Fläche des maximalen, senkrecht zu der Längsrichtung L der Katheterleitung 2 orientierten Querschnitts (der von dem Durchmesser dv1 bestimmt wird) des ersten Ventilelements 24 ist.

Um die Ventilelemente 24, 25 in Längsrichtung L der Katheterleitung 2 in dem ersten Kanal 15 in definierter Weise wahlweise hin- und herschieben zu können, weist der Ballonkatheter 1 eine Kraftübertragungseinrichtung 11 auf (vgl. auch die Figuren 4, 4a). Sie umfasst in dem Ausführungsbeispiel mehrere Drahtabschnitte 31 - 33. An dem distalen Längsende des ersten Drahtabschnittes 31 ist das Ventilelement 24 befestigt (in dem Beispiel angelötet). Das hohlzylindrische Ventilelement 25 ist auf den Drahtabschnitt 31 aufgeschoben und in an das Ventilelement 24 angrenzender Lage befestigt (in dem Beispiel angelötet). An seinem proximalen Längsende ist der erste Drahtabschnitt 31 fest mit dem distalen Längsende des Drahtabschnittes 32 verbunden (bspw. verlötet), und über die Verbindung ist zur Stabilisierung eine Hülse 34 geschoben und festgelegt. Der Drahtabschnitt 32 ist an seinem proximalen Längsende (nicht näher dargestellte Weise) fest mit dem distalen Längsende eines Drahtabschnittes 33 verbunden, an dessen proximalem Längsende ein Griffstück 35 fest angebracht ist. Die Kraftübertragungseinrichtung 11 erstreckt sich von dem ersten Ventilelement 24 in proximaler Richtung, in ihrer Längsrichtung verschiebbar, durch den ersten Kanal 15 und weiter durch den Fluiddurchlass 23 und dessen distalen Abzweig 36 durch die Anschlusseinrichtung 6 hindurch bis zu dem Griffstück 35. Die Länge der Drahtabschnitte 31 - 33 ist so gewählt, dass das Verschiebungs-Bewegungsspiel des Griffstückes 35 ausreicht, um die Ventilelemente 24, 25 wahlweise in den ersten Kanallängenabschnitt 27 oder in den zweiten Kanallängenabschnitt 29 zu verschieben, um das Ventil dadurch gezielt zu öffnen oder zu schließen. Figur 5 zeigt, dass sich der Fluiddurchlass 23 an seinem proximalen Ende neben distalen Abzweig 36 in einen seitlichen, zu dem Anschluss 7 führenden Abzweig 37 verzweigt. Ausgehend von dem Anschluss 9 erstreckt sich ein Röhrchen durch die Anschlusseinrichtung 6 bis in den zweiten Kanal 16 der Katheterleitung hinein. In den Anschluss 9 ist eine Dichtung eingesetzt.

In dem Beispiel, d. h. nicht notwendig, besitzen die Ventilelemente 24, 25 jeweils einen äußeren Durchmesser dv1 bzw. dv2 von 0,55 mm; der erste Kanal 15 besitzt im ersten Kanallängenabschnitt 27 einen Nenndurchmesser von 0,53 mm (Toleranz + 0,07 mm) und im zweiten Kanallängenabschnitt einen Nenndurchmesser von 0,45 mm (Toleranz + 0,05 mm). Bei dem Ausführungsbeispiel besitzt der Drahtabschnitt 31 einen Durchmesser von 0,3 mm und der Drahtabschnitt 32 einen Durchmesser von 0,5 mm. Es versteht sich aber, dass auch abweichende geometrische Maße und Maß-Verhältnisse möglich sind.

In der ungefähren Mitte des Toleranzfeldes besitzt der Kanaldurchmesser dk1 einen Wert von ca. 0,56 mm, der somit geringfügig größer als die sich entsprechenden Durchmesser dv1 und dv2 ist. Dies bedeutet, dass in einer Betrachtung senkrecht zur Längsrichtung L der Katheterleitung 2 die Fläche des Hohlquerschnitts in dem ersten Kanallängenabschnitt 27 im Beispiel größer als die Fläche des maximalen, senkrecht zur Längsrichtung L der Katheterleitung orientierten Querschnitts des ersten Ventilelements 24 ist. Alternativ wäre denkbar, dass dk1 gleich groß oder etwas kleiner als die sich entsprechenden Durchmesser dv1 und dv2 ausfällt, solange ein zum Befüllen des Ballons 3 mit Druck zugeführtes Gas (vorzugsweise Luft) innerhalb des ersten Kanallängenabschnittes 27 noch an den Ventilelementen 24, 25 vorbeiströmen kann, ggf. bei geringer elastischer Aufweitung der Katheterleitung 2.

Mit Bezug auf die in den Figuren 4, 4a gezeigte Kraftübertragungseinrichtung 11, die man auch als Ventilschieber bezeichnen könnte, wurde schon die Anbringung ihres Drahtabschnittes 31 an den Ventilelementen 24, 25 und die Verbindung zwischen den Drahtabschnitten 31 und 32 beschrieben. An seinem proximalen Längsende ist der Drahtabschnitt 32 (auf nicht näher dargestellte Weise) fest mit dem dritten Drahtabschnitt 33 verbunden, an dessen proximalem Längsende das Griffstück 35 befestigt ist. Figur 5 zeigt, wie die Kraftübertragungseinrichtung 11 in die Anschlusseinrichtung 6 eingesetzt und darin in dem Fluiddurchlass 23 und durch deren distalen Abzweig 36 hindurch in ihrer Längsrichtung verschieblich geführt ist. In dem Beispiel besitzt der zweite Anschluss 8 ein Außengewinde, zu dem ein Innengewinde in dem Griffstück 35 passt. Damit lässt sich das Griffstück 35 in seiner distalen Endlage sichern. Der distale Auslauf des Innengewindes bildet einen ersten Anschlag 38, und die proximale Stirnfläche des zweiten Anschlusses 8 bildet einen ersten Gegenanschlag 39. Wenn das Griffstück 35 bspw. aus der in Figur 5 gezeigten Position in distaler Richtung zu der Anschlusseinrichtung 6 verschoben wird, tritt der Anschlag 38 schließlich in Verschieberichtung gegen den Gegenanschlag 39. In dieser distalen Endposition der Kraftübertragungseinrichtung 11 begrenzen der erste Anschlag 38 und der erste Gegenanschlag 39 den Verschiebeweg formschlüssig. Die Länge der Kraftübertragungseinrichtung 11, bzw. die Länge der Drahtabschnitte 31 - 33, ist so gewählt, dass, wenn sich das Griffstück 35 in seiner distalen Endposition befindet, das erste Ventilelement 24 vollständig und das zweite Ventilelement 25, je nach Ausführung, vollständig oder zumindest mit einem Längenteilabschnitt in dem zweiten Kanallängenabschnitt 29 befindet, so dass dort der erste Kanal 15 gegen Durchtritt von Fluid abgedichtet, d. h. verschlossen ist. In dem Beispiel ist an der Kraftübertragungseinrichtung 11 ein zweiter Anschlag 43 befestigt. In dem Beispiel handelt es sich um eine Kugel mit einer Durchgangsöffnung, die auf den Drahtabschnitt 3 aufgeschoben und darauf befestigt, bspw. verlötet, ist, so dass keine Relativbewegung in der Verschieberichtung der Kraftübertragungseinrichtung 11 möglich ist. An der Anschlusseinrichtung 6 ist ein zweiter Gegenanschlag 44 befestigt. Im Beispiel handelt es sich um eine Silikondichtung 40, an die sich proximal ein Verschlussstopfen 41 und eine weitere Silikondichtung 42 als sandwichartige Anordnung anschließen, die eine zentrale Durchgangsöffnung 45 für den Drahtabschnitt 33 belässt. Besagte sandwichartige Anordnung ist in dem Gehäuse der Anschlusseinrichtung 6 festgelegt, so dass in der Verschieberichtung der Kraftübertragungseinrichtung 11 keine Relativbewegung möglich ist. Der Querschnitt (bzw. Durchmesser) des zweiten Anschlages 43 ist größer als der Durchmesser der Durchgangsöffnung 45 gewählt. Wird das Griffstück 35 in proximaler Richtung von der Anschlusseinrichtung 6 weg bewegt, tritt schließlich in der in Figur 5 gezeigten Verschiebeposition der zweite Anschlag 43 formschlüssig gegen den Gegenanschlag 44. Die Position des zweiten Anschlags 43 an der Kraftübertragungseinrichtung 11 ist so gewählt, dass sich in dieser proximalen Endposition die Ventilelemente 24 und 25 in dem ersten Kanallängenabschnitt 27 befinden, so dass der erste Kanal 15 zum Hindurchströmen von Fluid (vorzugsweise Luft) geöffnet ist. Aus den Figuren wird deutlich, dass es die Erfindung ermöglicht, dass sich der Übergang 28 zwischen den beiden Kanallängenabschnitten 27, 29 außerhalb der Anschlusseinrichtung 6 in einem gewünschten Abstand in distaler Richtung befinden kann. Dieser Abstand kann bspw. wenige Zentimeter oder ggf. nur Millimeter betragen, könnte aber bei Bedarf auch größer sein.

Bei dem in den Figuren gezeigten Ausführungsbeispiel ist die Katheterleitung 2 einstückig aus elastischem Kunststoff, in dem Beispiel aus Polytetrafluorethylen (PTFE) hergestellt.

In dem Ausführungsbeispiel umfasst der Ballonkatheter 1 eine Knickschutzhülse 46, die in einem kurzen Stück auf das distale Längsende der Anschlusseinrichtung 6 unter Erzielung einer Haltekraft aufgesteckt ist. Die Knickschutzhülse 46 ist aus blickdichtem, elastischem Material hergestellt und ummantelt lose einen Längenabschnitt der Katheterleitung 2. In distaler Richtung erstreckt sich die Knickschutzhülse 46 bis in den zweiten Kanallängenabschnitt 29. Sie weist außenseitig eine erste, als Farbring in einer ersten Farbe (im Beispiel grün) gestaltete Markierung 47 auf. In Bezug auf den Übergang 28 (dabei handelt es sich um den Querschnittsübergang zwischen dem ersten und dem zweiten Kanallängenabschnitt) ist die Markierung 47 mit einem Abstand von mehreren Millimetern oder Zentimetern in proximaler Richtung angeordnet. Eine zweite Markierung 48, bei der es sich im Beispiel um einen roten Farbring handelt, ist unmittelbar an der Außenseite der Katheterleitung 2 angebracht. Gemäß den Figuren 1a - 3a befindet sich die zweite Markierung 48 in distalem Abstand von dem Übergang 28 und dabei innerhalb des Längenabschnittes der Katheterleitung 2, der in dem in den Figuren 1 - 3 gezeigten Gebrauchszustand von der Knickschutzhülle 46 ummantelt wird. In diesem Gebrauchszustand ist daher die zweite Markierung 48 nicht sichtbar, und daher in den Figuren 1 - 3 ebenso wie die Ventilelemente 24, 25 nur zur positionsmäßigen Orientierung gestrichelt angedeutet. Erst wenn die Knickschutzhülse 46 entfernt wird, wird die zweite Markierung 48 erkennbar.

Bei dem Ausführungsbeispiel ist vorgesehen, dass sich innerhalb der Katheterleitung 2, parallel zu dem ersten hohlen Kanal 15, ein zweiter hohler Kanal 16 von vergleichsweise größerem Durchmesser erstreckt. Der zweite Kanal 16 erstreckt sich entlang der gesamten Länge der Katheterleitung 2 und verbindet den dritten Anschluss 9 der Anschlusseinrichtung 6 mit einer Öffnung 49 (diese wurde auch als 22 bezeichnet) der Katheterleitung 2 an ihrem distalen Ende 5. Der zweite Kanal 16 ermöglicht das Hindurchführen eines Führungsdrahtes 50 (wie noch nachfolgend erläutert). Außerdem ermöglicht er das Hindurchleiten von Kontrastmittel, wozu bei Bedarf an der Anschlusseinrichtung 6 ein Kontrastmittelansatz 13 angebracht werden kann.

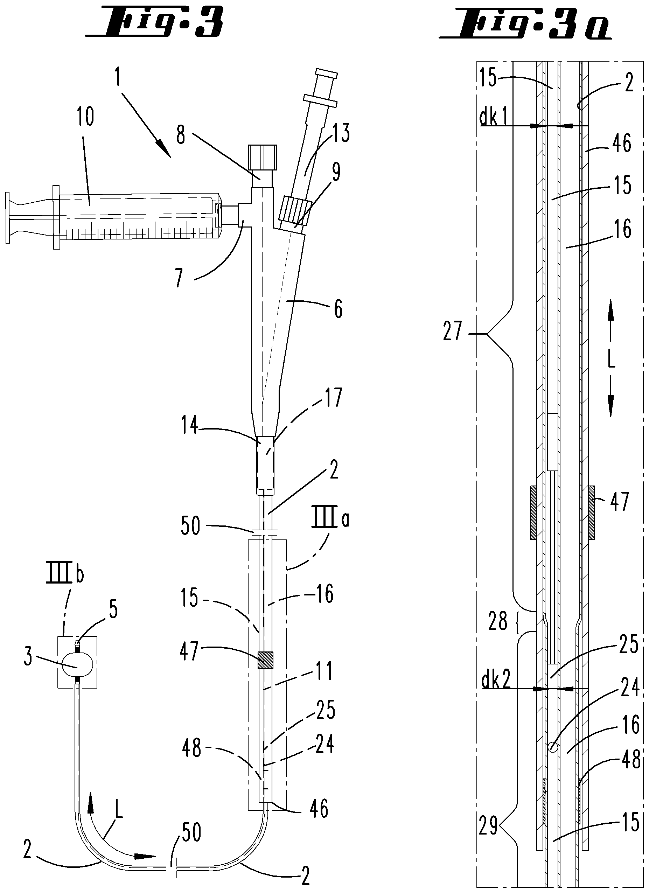

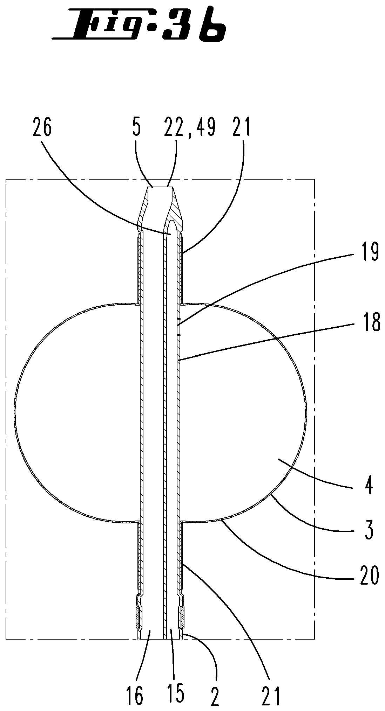

In Figur 1 ist der Ballon 3 entleert, so dass die Ballonhülle schlank an dem Führungsdraht 2 anliegt. Die Kraftübertragungseinrichtung 11 (d. h. der Ventilschieber) befindet sich in seiner proximalen Endposition, so dass das die Ventilelemente 24, 25, die Kanallängenabschnitte 27, 29 und den Übergang 218 umfassende Ventil geöffnet ist und somit der erste Kanal 15 von Fluid (in dem Beispiel Luft) durchströmt werden kann. Um den Ballon 3 mit Druckluft zu befüllen, ist an dem ersten Anschluss 7 eine Spritze 10 als Befüllungseinrichtung angeschlossen. Bei dem in Figur 2 gezeigten späteren Gebrauchszustand wurde der Kolben der Spritze eingedrückt und dadurch der Ballon 3 aufgepumpt. Bei dem Gebrauchszustand gemäß Figur 3 wurde die Kraftübertragungseinrichtung 11 (der Ventilschieber) in seine distale Endposition bewegt, so dass der erste Kanal 15 nun geschlossen ist und die Druckluft aus dem Ballon 3 nicht mehr zurückströmen kann. Die radiale, elastische Aufweitung der Wandung der Katheterleitung in dem zweiten Kanallängenabschnitt zufolge der Ventilelemente 24, 25 ist so gering, dass sie in der Figur nicht erkennbar ist. Wird, ausgehend von Figur 3, das Griffstück wieder in seine proximale Endposition gezogen, wird das besagte Ventil in dem ersten Kanal 15 wieder geöffnet, sodass die Druckluft aus dem Ballon 3 zurückströmen kann.

Mit Bezug auf die Figuren 8 - 11 werden exemplarisch verschiedene denkbare Gebrauchszustände des in den Figuren 1 -7 dargestellten Ballonkatheters 1 während exemplarischer Anwendungen dargestellt.

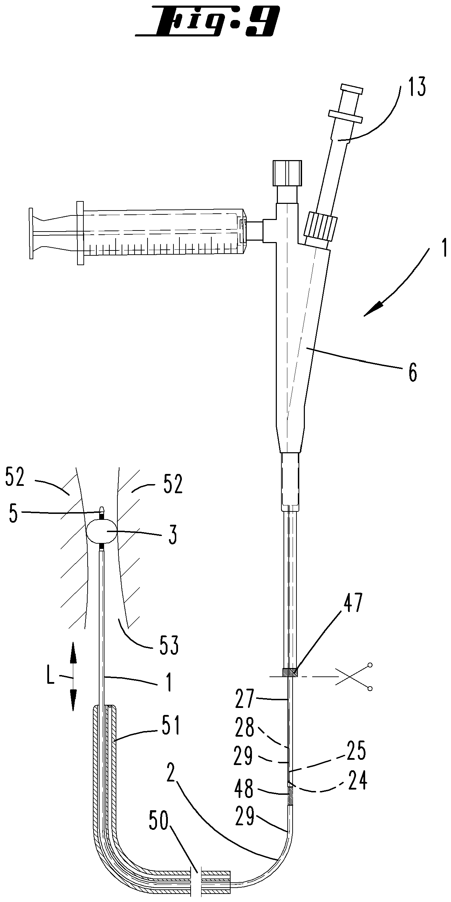

Bei dem in Figur 8 gezeigten Gebrauchszustand wurde zunächst ein Führungsdraht 12 durch den Arbeitskanal eines ersten, in seinem Querschnitt vergleichsweise dicken Endoskops 51 in distaler Richtung hindurchgeführt, bis sich das distale Längsende des Führungsdrahtes 50 an einer gewünschten Position in einem schematisch durch Begrenzungswände 52 angedeuteten Hohlraum 53 in einem menschlichen oder tierischen Körper befindet. Entlang des vorgeschobenen Führungsdrahtes 50 wurde der Ballonkatheter 1 mit drucklosem, d. h. schlankem, Ballon 3 durch den Arbeitskanal des dicken Endoskops 51 geführt, bis sich der Ballon 3 an einer gewünschten Position in dem Hohlraum 53 befindet. Der Führungsdraht 12 kann dazu durch den zweiten Kanal 16 der Katheterleitung 2 geleitet werden. In dieser Position wurde das an der Spritze 10 befindliche Fluid (in dem Beispiel Luft) durch den ersten Kanal 15 in der zuvor beschriebenen Weise in den Ballon 3 geleitet, wodurch sich dieser, wie Figur 8 zeigt, elastisch aufweitet und sich dadurch zwischen den Begrenzungswänden 52 verspannt. Der Ballon 3 verankert dadurch den Ballonkatheter 1 im Bereich seines distalen Längsendes 5, so dass ein ungewolltes Verschieben des Ballonkatheters 1, insbesondere in seiner Längsrichtung L, wirksam verhindert wird. Um ein Zurückströmen der Luft aus dem Ballon 3 zu verhindern, wurde das Griffstück 35 in seine distale Endposition vorgeschoben und dort mittels eines Gewindes an der Anschlusseinrichtung 6 gesichert. Figur 9 zeigt, dass die Querschnittsabmessungen der Anschlusseinrichtung 6 größer sind als der Querschnitt des Arbeitskanals im Endoskop 51, so dass das Endoskop 51 nicht in proximaler Richtung über die Anschlusseinrichtung 6 zurückgezogen werden kann. Um dennoch zu ermöglichen, dass das Endoskop 51 proximal von dem Ballonkatheter 1 abgezogen werden kann, besteht nach dem Entfernen des Führungsdrahtes 12 die Möglichkeit, den Ballonkatheter 1 an seiner Markierung 47 zu durchtrennen, bspw. zu durchschneiden. Den resultierenden Zustand zeigt Figur 9. Der sich an die Markierung 47 distal anschließende Längenabschnitt der Knickschutzhülle 46 umgibt die Katheterleitung 2 nur lose, so dass sich dieser Längenabschnitt frei entlang der Katheterleitung 2 verschieben lässt. In Figur 9 wurde dieser bewegliche Abschnitt der Knickschutzhülle 46 schon entfernt. Der Übergang 28 befindet sich nach wie vor in dem mit dem Ballon 3 verbundenen Längenabschnitt der Katheterleitung 2, und die Ventilelemente 24, 25 befinden sich nach wie vor in dem zweiten Kanallängenabschnitt 29. Dies bewirkt, dass der Ballon 3 aufgepumpt bleibt und seine Verankerungswirkung beibehält.

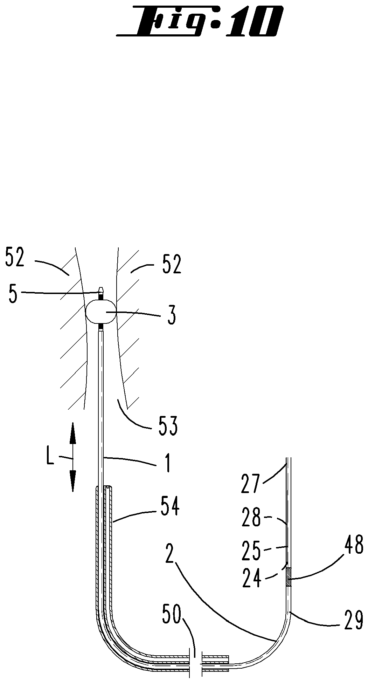

Wie Figur 9 auch zeigt, wird die zweite Markierung 48 erst nach dem Abziehen des losen Abschnittes der Knickschutzhülle 46 sichtbar. Dies verringert das Risiko einer Fehlbedienung. Nach dem Abtrennen der Anschlusseinrichtung 6 kann, wie ein Vergleich der Figuren 9 und 10 zeigt, das erste Endoskop 51 in proximaler Richtung von der Katheterleitung 2 abgezogen und bspw. gegen ein in Figur 10 gezeigtes, vergleichsweise schlankeres Endoskop 54 ersetzt werden, durch dessen Arbeitskanal die Katheterleitung 2 wieder hindurchgeführt werden kann. Der aufgepumpte Ballon 3 behält dabei seine Position.

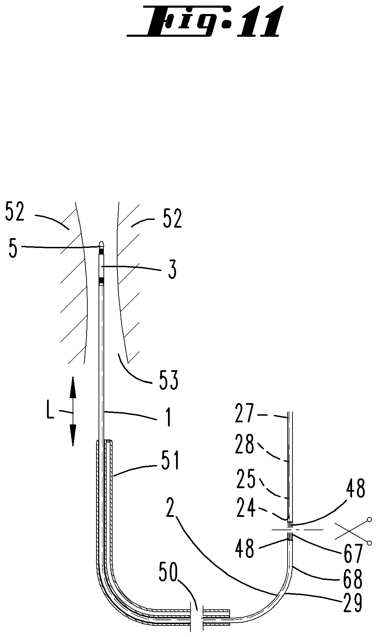

Erst wenn, wie in Figur 11 dargestellt ist, die Katheterleitung 2 nachfolgend an der Markierung 48 durchtrennt, bspw. zerschnitten, wird, werden die Ventilelemente 24, 25 von der mit dem Ballon 3 verbundenen Katheterleitung 2 abgetrennt. Dies bewirkt, dass das Fluid rasch aus dem Ballon 3 austreten kann, so dass der Ballon 3 schnell kontrahiert und der Ballonkatheter 1 nun aus dem Hohlraum 53 und bei Bedarf aus dem Endoskop 54 in proximaler Richtung zurückgezogen werden kann.

Aus der vorangehenden Beschreibung wird deutlich, dass der Ballon 3 bei der in den Figuren gezeigten exemplarischen Verwendung bis zum Abschneiden der Ventilelemente 24, 25, d. h. solange der Ballon 3 aufgepumpt und dadurch expandiert ist, zwischen den Begrenzungswänden 52 eingeklemmt bleibt und folglich auch während des Endoskop-Austausches unverändert in der in dem Hohlraum 53 gewünschten Position verbleibt. Zufolge dieser Verankerung des Ballons 3 kann erreicht werden, dass sich der Ballonkatheter 1 während des Austausches der Endoskope 51, 54 nicht in Längsrichtung L verschiebt. Erst eine Kontraktion des Ballons 3 beseitigt diese Haltewirkung, so dass die Lagefixierung aufgehoben wird und der Ballon 3 zwischen den Begrenzungswänden 52 heraus und durch das zuletzt verwendete Endoskop 54 zurückgezogen werden kann. Es versteht sich aber, dass auch andere Möglichkeiten zur Anwendung bestehen. So ermöglicht es zum Beispiel der erfindungsgemäße Ballonkatheter in seinem noch unzerschnittenen Ausgangszustand, dass die Kraftübertragungseinrichtung 11 (der Ventilschieber) bei Bedarf mehrfach hin- und hergeschoben wird, um im Zusammenwirkung mit der Befüllungseinrichtung 56 (Spritze 10) den Ballon 3 je nach gewünschter Anwendung mehrfach zu expandieren und zu kontrahieren. Die Aufbrüche 50 in den Figuren stellen keine tatsächlichen Trennstellen dar, sondern dienen nur symbolisch zur verkürzten Darstellung der Katheterleitung 2, deren Länge deutlich größer als dargestellt sein kann.

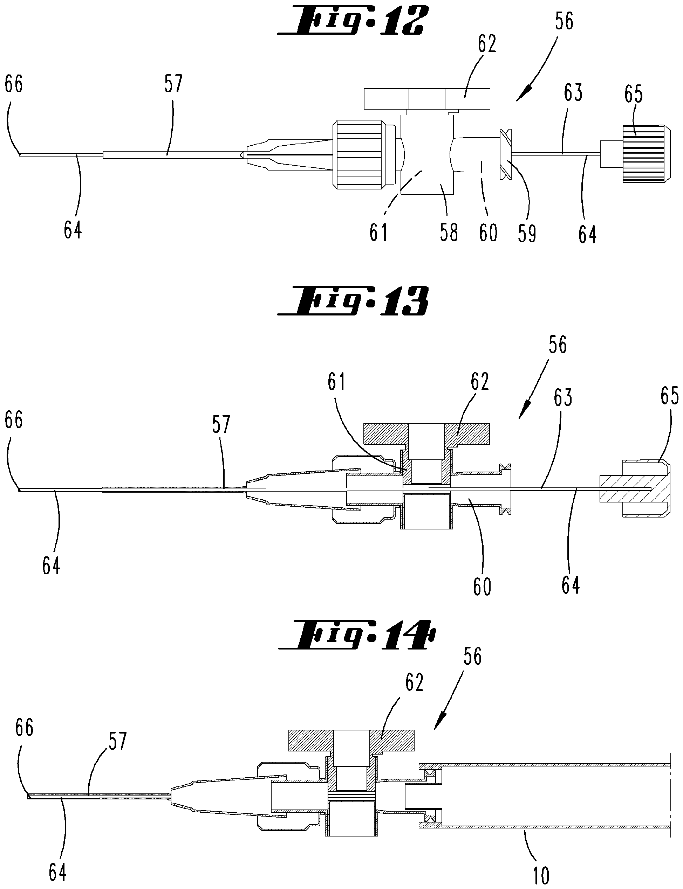

Für gewisse Anwendungen kann es auch vorteilhaft sein, wenn sich der Ballon 3 auch nach dem Abtrennen der Ventilelemente 24, 25 nochmals mit Luft befüllen und dadurch expandieren lässt. Zum Beispiel kann eine solche Wiederbefüllung des Ballons 3 unmittelbar wieder an einer Position zwischen den Begrenzungswänden 52 des Körperhohlraums 53 erfolgen. Möglich wäre aber auch eine Wiederbefüllung des Ballons 3 an einem anderen Ort in einem Körper. Eine dazu geeignete Befüllungseinrichtung 56, die im Hinblick auf diese bevorzugte Funktion auch als Wiederbefüllungseinrichtung zu bezeichnen ist, wird zunächst mit Bezug auf die Figuren 12 - 14 beschrieben, wobei dort die Darstellung gegenüber den vorangehenden und nachfolgenden Figuren vergrößert ist. Die Befüllungseinrichtung 56 umfasst eine Hohlnadel 57 und ein an sie dichtend anschließendes Gehäuse 58, das in dem Beispiel aus Kunststoff besteht. Die Befüllungseinrichtung 56 besitzt auf der zu der Hohlnadel 57 gegenüberliegenden, proximalen Seite einen Anschluss 59, der den lösbaren Anschluss einer Spritze 10 (vgl. Figur 14) ermöglicht. Im Inneren des Gehäuses 58 erstreckt sich ein hohler Fluiddurchlass 60, der distal mit der Hohlnadel 57 und proximal mit dem Anschluss 59 in fluidischer Verbindung steht. In das Gehäuse 58 bzw. in dessen Fluiddurchlass 60 ist ein Absperrventil 61 eingesetzt, dessen Ventilkörper sich mittels eines Drehgriffes 62 wahlweise in eine Drehstellung drehen lässt, in der eine Durchgangsöffnung in dem Ventilkörper eine von Fluid (vorzugsweise Luft) durchströmbare Verbindung zwischen den auf beiden Seiten des Absperrventils 61 (also davon und dahinter) befindlichen Abschnitten des Fluiddurchlasses 60 bildet, oder in eine andere Drehstellung, in der der Ventilkörper diese beiden Abschnitte voneinander dichtend trennt. Der Fluiddurchlass 60 ist somit mittels der Absperrventiles 61 wahlweise zum Durchlass von Fluid aus der Spritze 10 öffenbar oder zur Verhinderung von Fluiddurchtritt verschließbar. Auf das Absperrventil 61 könnte jedoch in dem Beispiel verzichtet werden, wenn sich die Spritze 10 dichtend an das Gehäuse 58 anschließen lässt und der Spritzenkolben im Spritzengehäuse auch abgedichtet ist. Der Außendurchmesser der Hohlnadel 57 ist nur um wenige Hundertstel Millimeter, also nur geringfügig, größer als der Innendurchmesser des ersten Kanals 15 der elastischen Katheterleitung 2. In den Figuren 12, 13 ist ein Mandrin 63 durch die Befüllungseinrichtung 56 hindurchgeführt. Er umfasst einen Drahtabschnitt 64 und einen proximalen Griffabschnitt 65. Der Drahtabschnitt 64 erstreckt sich durch den Anschluss 59, den Fluiddurchlass 60, das geöffnete Absperrventil 61 und die Hohlnadel 57 und steht mit seinem distalen Längsende 66 über die Spitze der Hohlnadel 57 hervor. In Figur 14 ist der Mandrin 63 aus der Befüllungseinrichtung 56 entnommen und eine Spritze 10 dichtend an den Anschluss 59 angeschlossen.

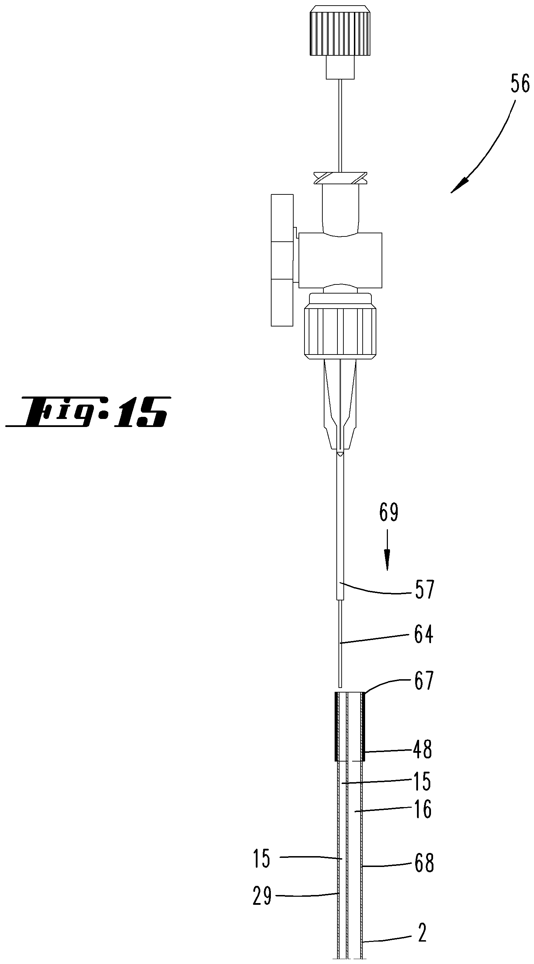

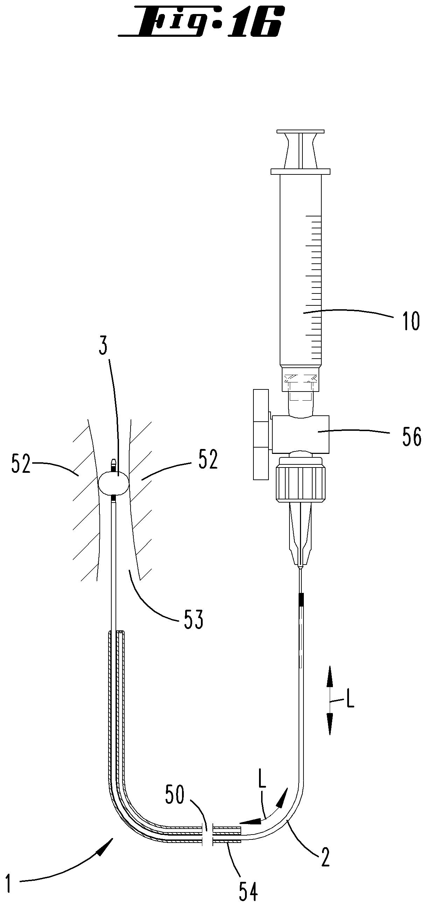

Die Figuren 15, 16 zeigen exemplarisch, wie sich bspw. ausgehend von dem Gebrauchszustand des Ballonkatheters 1 gemäß Figur 11 die Befüllungseinrichtung 56 zur erneuten Befüllung bzw. Expansion des Ballons 3 verwenden lässt. Dazu zeigt Figur 15 das proximale Längsende 67 eines mit dem Ballon 3 noch fest verbundenen Längenabschnittes 68 der Katheterleitung 2, das im Bereich der Markierung 48 beim Abtrennen gebildet wurde. Dort liegen der erste Kanal 15 und der zweite Kanal 16 stirnseitig frei. Figur 15 zeigt, dass die Befüllungseinrichtung 56 mit dem darin eingesetzten Mandrin 63 in Bewegungsrichtung des Pfeiles 69 an dem proximalen Längsende 67 in den ersten Kanal 15 eingeführt wird. Der Drahtabschnitt 64, dessen Durchmesser kleiner als der Außendurchmesser der Hohlnadel 57 ist, dient als Einfädelhilfe. Die Hohlnadel 57 wird unter Aufbringung einer Längskraft in den ersten Kanal 15 eingesteckt, der dabei zufolge der beschriebenen Durchmesserverhältnisse etwas elastisch aufgeweitet wird, so dass er die Hohlnadel 57 schließlich unter Erzielung einer Steck-Klemmverbindung dichtend umschließt. Figur 16 zeigt, dass nach dem Einstecken der Hohlnadel 57 in den ersten Kanal 15 der Mandrin 63 entfernt und eine Spritze 10 an den Anschluss 59 angeschlossen werden kann. Figur 16 zeigt einen Gebrauchszustand, bei dem die Luft aus der Spritze 10 schon mittels der Befüllungseinrichtung 56 in den ersten Kanal 15 der Katheterleitung 2 und durch den ersten Kanal 15 hindurch in den Ballon 3 gepumpt wurde, der somit erneut befüllt und expandiert worden ist, so dass eine gewünschte Klemmwirkung bzw. Lagefixierung zwischen den Begrenzungswänden 52 des Körperhohlraumes 53 erneut erreicht werden kann. Um diesen Zustand (auch mit der Möglichkeit, die Spritze 10 abzunehmen) zu erhalten, kann das Absperrventil 61 in seine Geschlossenstellung gedreht werden. Um den Ballon erneut entlüften bzw. kontrahieren zu können, so dass er sich aus dem Hohlraum 53 entfernen lässt, besteht die Möglichkeit, dass die Hohlnadel 57 aus der Katheterleitung 2 proximal gezogen wird oder dass das Absperrventil geöffnet wird. Der Ballon 3 ist in der in den Figuren 1b und 2b geschnitten gezeigten Weise an der Katheterleitung 2 angebracht bzw. ausgebildet, d. h. die Katheterleitung 2 erstreckt sich durch das Innere des Ballons 3. In ungeschnittenen Darstellungen des Ballons 3 (wie bspw. in Figur 16) ist in den Figuren der durch den Ballon 3 hindurchführende Abschnitt der Katheterleitung 2 zur besseren Übersicht nicht mit dargestellt, obwohl die Ballonhülle auch bspw. aus einem durchscheinenden Material bestehen kann.

**Bezugszeichenliste:**

| | | | |
|---|---|---|---|
| 1 | Ballonkatheter | 25 | zweites Ventilelement |
| 2 | Katheterleitung | 26 | distales Längsende |
| 3 | Ballon | 27 | erster Kanallängenabschnitt |
| 4 | Balloninnenraum | 28 | Übergang |
| 5 | distales Längsende | 29 | zweiter Kanallängenabschnitt |
| 6 | Anschlusseinrichtung | 30 | Wandung |
| 7 | erster Anschluss | 31 | Drahtabschnitt |
| 8 | zweiter Anschluss | 32 | Drahtabschnitt |
| 9 | dritter Anschluss | 33 | Drahtabschnitt |
| 10 | Spritze | 34 | Hülse |
| 11 | Kraftübertragungseinrichtung | 35 | Griffstück |
| 12 | Führungsdraht | 36 | distaler Abzweig |
| 13 | Kontrastmittelansatz | 37 | seitlicher Abzweig |
| 14 | Verbindung | 38 | erster Anschlag |
| 15 | erster Kanal | 39 | erster Gegenanschlag |
| 16 | zweiter Kanal | 40 | Silikondichtung |
| 17 | proximales Längsende | 41 | Verschlussstopfen |
| 18 | Wandung | 42 | Silikondichtung |
| 19 | Durchgangsbohrung | 43 | zweiter Anschlag |
| 20 | Ballonhülle | 44 | zweiter Gegenanschlag |
| 21 | Drahtwicklungen | 45 | Durchgangsöffnung |
| 22 | stirnseitige Öffnung | 46 | Knickschutzhülse |
| 23 | Fluiddurchlass | 47 | erste Markierung |
| 24 | erstes Ventilelement | 48 | zweite Markierung |
| 49 | Öffnung | 67 | proximales Längsende |
| 50 | Führungsdraht | 68 | Längenabschnitt |
| 51 | Endoskop | 69 | Pfeil |
| 52 | Begrenzungswände | | |
| 53 | Hohlraum | | |
| 54 | Endoskop | | |
| 55 | Aufbruch | | |
| 56 | Befüllungseinrichtung | | |
| 57 | Hohlnadel | | |
| 58 | Gehäuse | | |
| 59 | Anschluss | | |
| 60 | Fluiddurchlass | | |
| 61 | Absperrventil | | |
| 62 | Drehgriff | | |
| 63 | Mandrin | | |
| 64 | Drahtabschnitt | | |
| 65 | Griffabschnitt | | |
| 66 | distales Längsende | | |

## Patentansprüche

1. Ballonkatheter (1), umfassend eine Katheterleitung (2), einen daran angebrachten, dehnbaren Ballon (3), der einen Balloninnenraum (4) berandet, und eine mit der Katheterleitung (2) verbundene Anschlusseinrichtung (6), die zumindest einen ersten Anschluss (7) zur Zufuhr von Fluid, insbesondere von Luft, zum Befüllen des Ballons (3) aufweist, wobei sich in der Katheterleitung (2) zumindest ein erster Kanal (15) erstreckt, der an seinem proximalen Längsende (17) mit dem ersten Anschluss (7) in fluidischer Verbindung steht und der an seinem distalen Längsende (26) mit dem Balloninnenraum (4) in fluidischer Verbindung steht, wobei der erste Kanal (15) einen ersten Kanallängenabschnitt (27) und einen sich daran in distaler Richtung mit einem Übergang (28) anschließenden zweiten Kanallängenabschnitt (29) aufweist, wobei die Katheterleitung (2) zumindest entlang dieser Kanallängenabschnitte (27, 29) und entlang des Übergangs (28) aus elastischem Material hergestellt ist, wobei der Ballonkatheter (1) zumindest ein erstes, insbesondere als Kugel ausgebildetes, Ventilelement (24) aufweist, das in den ersten Kanal (15) eingesetzt ist, wobei, insbesondere bei jeweils entspannter Wandung der Katheterleitung (2), die Fläche des Hohlquerschnitts des ersten Kanals (15) in dem zweiten Kanallängenabschnitt (29) kleiner als die Fläche des Hohlquerschnitts des ersten Kanals (15) in dem ersten Kanallängenabschnitt (27) und kleiner als die Fläche des senkrecht zur Längsrichtung (L) der Katheterleitung (2) orientierten Querschnitts des ersten Ventilelements (24) ist, und wobei der Ballonkatheter (1) eine Kraftübertragungseinrichtung (11) aufweist, **dadurch gekennzeichnet, dass** die Kraftübertragungsvorrichtung (11) an dem ersten Ventilelement (24) befestigt ist und sich von dem ersten Ventilelement (24) in proximaler Richtung durch den ersten Kanal (15) und weiter durch die Anschlusseinrichtung (6) hindurch bis zu einem Griffstück (35) erstreckt, wobei mittels Bewegung des Griffstückes (35) zu der Anschlusseinrichtung (6) das erste Ventilelement (24) aus dem ersten Kanallängenabschnitt (27) in den zweiten Kanallängenabschnitt (29) unter dortiger elastischer Dehnung der Wandung (30) der Katheterleitung (2) bewegbar ist.

2. Ballonkatheter (1) nach Anspruch 1, **dadurch gekennzeichnet, dass** die Fläche des Hohlquerschnitts des ersten Kanals (15) an jeder Stelle in dem zweiten Kanallängenabschnitt (29) kleiner als die Fläche des Hohlquerschnitts des ersten Kanals (15) an jeder Stelle in dem ersten Kanallängenabschnitt (27) und kleiner als die Fläche des größten senkrecht zur Längsrichtung (L) der Katheterleitung (2) orientierten Querschnitts des ersten Ventilelements (24) ist.

3. Ballonkatheter (1) nach einem oder mehreren der vorangehenden Ansprüche, **dadurch gekennzeichnet, dass**, insbesondere bei jeweils entspannter Wandung (30) der Katheterleitung (2), der Hohlquerschnitt des ersten Kanals (15) in dem ersten Kanallängenabschnitt (27) einheitlich oder im Wesentlichen einheitlich ist, wobei im Wesentlichen einheitlich bedeutet, dass etwaige Größenunterschiede nur im einstelligen Prozentbereich liegen, und der hiervon flächenmäßig abweichende Hohlquerschnitt des ersten Kanals (15) in dem zweiten Kanallängenabschnitt (29) darin ebenfalls einheitlich oder im Wesentlichen einheitlich ist, wobei im Wesentlichen einheitlich bedeutet, dass etwaige Größenunterschiede nur im einstelligen Prozentbereich liegen.

4. Ballonkatheter (1) nach Anspruch 1, **dadurch gekennzeichnet, dass**, insbesondere bei entspannter Wandung (30) der Katheterleitung (2), die Fläche des Hohlquerschnitts des ersten Kanals (15) in dem ersten Kanallängenabschnitt (27) größer als oder gleich groß wie oder kleiner als die Fläche des Querschnitts des ersten Ventilelements (24) ist.

5. Ballonkatheter (1) nach einem oder mehreren der vorangehenden Ansprüche, **dadurch gekennzeichnet, dass**, insbesondere bei jeweils entspannter Wandung (30) der Katheterleitung (2), der erste Kanal (15) in dem ersten Kanallängenabschnitt (27) einen kreisförmigen Hohlquerschnitt mit einem in dem ersten Kanallängenabschnitt (27) einheitlichen ersten Kanaldurchmesser (dk1) und in dem zweiten Kanallängenabschnitt (29) einen kreisförmigen Hohlquerschnitt mit einem in dem zweiten Kanallängenabschnitt (29) einheitlichen zweiten Kanaldurchmesser (dk2) besitzt, wobei der zweite Kanaldurchmesser (dk2) kleiner als der erste Kanaldurchmesser (dk1) und kleiner als der Durchmesser des als Kugel oder Halbkugel ausgebildeten ersten Ventilelements (24) ist.

6. Ballonkatheter (1) nach einem oder mehreren der vorangehenden Ansprüche, **dadurch gekennzeichnet, dass**, insbesondere bei jeweils entspannter Wandung (30) der Katheterleitung (2), im ersten Kanallängenabschnitt (27) der erste Kanaldurchmesser (dk1) größer als oder gleich groß wie oder kleiner als der Durchmesser (dv1) des als Kugel oder Halbkugel ausgebildeten ersten Ventilelements (24) ist.

7. Ballonkatheter (1) nach einem oder mehreren der vorangehenden Ansprüche, **dadurch gekennzeichnet, dass** sich in dem ersten Kanal (15) in dem Übergang (28) zwischen dem ersten Kanallängenabschnitt (27) und dem zweiten Kanallängenabschnitt (29) der Hohlquerschnitt des ersten Kanals (15) in distaler Richtung kontinuierlich, insbesondere konisch, verjüngt.

8. Ballonkatheter (1) nach einem oder mehreren der vorangehenden Ansprüche, **dadurch gekennzeichnet, dass** an der Kraftübertragungseinrichtung (11) ein erster Anschlag (38) befestigt oder ausgebildet ist und dass an der Anschlusseinrichtung (6) ein erster Gegenanschlag (39) befestigt oder ausgebildet ist und dass das Griffstück (35) zu der Anschlusseinrichtung (6) bis zu einer distalen Endposition vorschiebbar ist, bei deren Erreichen der erste Anschlag (38), die Verschiebung formschlüssig begrenzend, gegen den ersten Gegenanschlag (39) tritt und sich das erste Ventilelement (24) in dem zweiten Kanallängenabschnitt (29) befindet.

9. Ballonkatheter (1) nach einem oder mehreren der vorangehenden Ansprüche, **dadurch gekennzeichnet, dass** an der Kraftübertragungseinrichtung (11) ein zweiter Anschlag (43) befestigt oder ausgebildet ist und an der Anschlusseinrichtung (6) ein zweiter Gegenanschlag (44) befestigt oder ausgebildet ist und dass das Griffstück (35) von der Anschlusseinrichtung (6) bis zu einer proximalen Endposition wegziehbar ist, bei deren Erreichen der zweite Anschlag (43), die Ziehbewegung formschlüssig begrenzend, gegen den zweiten Gegenanschlag (44) tritt und sich das erste Ventilelement (24) in dem ersten Kanallängenabschnitt (27) befindet.

10. Ballonkatheter (1) nach einem oder mehreren der vorangehenden Ansprüche, **dadurch gekennzeichnet, dass** der Ballonkatheter (1) ein zweites, insbesondere zylinderförmiges, Ventilelement (25) aufweist, das an das erste Ventilelement (24) an dessen proximaler Seite angrenzt, wobei insbesondere vorgesehen ist, dass der Durchmesser (dv2) des zweiten Ventilelements (25) dem Durchmesser (dv1) des ersten Ventilelements (24) entspricht, wobei, wenn sich das Griffstück (35) in der distalen Endposition befindet, sich das zweite Ventilelement (25) ganz oder teilweise in dem zweiten Kanallängenabschnitt (29) des ersten Kanals (15) befindet.

11. Ballonkatheter (1) nach einem oder mehreren der vorangehenden Ansprüche, **dadurch gekennzeichnet, dass** das proximale Längsende des zweiten Kanallängenabschnittes (29) von der Anschlusseinrichtung (6) in distaler Richtung beabstandet ist.

12. Ballonkatheter (1) nach einem oder mehreren der vorangehenden Ansprüche, **dadurch gekennzeichnet, dass** die Außenoberfläche der Katheterleitung (2) zumindest entlang dem ersten und zweiten Kanallängenabschnitt (27, 29) und dem Übergang (28) aus Kunststoff, wie bspw. PTFE, oder aus Gummi besteht.

13. Ballonkatheter (1) nach einem oder mehreren der vorangehenden Ansprüche, **dadurch gekennzeichnet, dass** die Katheterleitung (2), zumindest in einem den ersten Kanallängenabschnitt (27), den Übergang (28) und den zweiten Kanallängenabschnitt (29) einschließenden Längenteilabschnitt, einstückig ausgebildet ist.

14. Ballonkatheter (1) nach einem oder mehreren der vorangehenden Ansprüche, **dadurch gekennzeichnet, dass** der Ballonkatheter (1) eine Knickschutzhülse (46) aufweist, die an der Anschlusseinrichtung (6) angebracht ist und die von dort lose einen Längenteilabschnitt der Katheterleitung, der sich von der Anschlusseinrichtung (6) in distaler Richtung bis in den zweiten Kanallängenabschnitt (29) erstreckt, ummantelt, und dass an der Knickschutzhülse (46) eine von außen sichtbare erste Markierung (47), insbesondere ein Ring in einer ersten Farbe, angebracht ist, wobei die erste Markierung (47) von dem distalen Längsende des ersten Kanallängenabschnitts (27) in proximaler Richtung beabstandet ist.

15. Ballonkatheter (1) nach einem oder mehreren der vorangehenden Ansprüche, **dadurch gekennzeichnet, dass** an der Katheterleitung (2) eine zweite Markierung (48), insbesondere ein Ring in einer zweiten Farbe, angebracht ist, die, wenn die Katheterleitung (2) freiliegt, von außen sichtbar ist, wobei die zweite Markierung (48) von dem proximalen Längsende des zweiten Kanallängenabschnitts (29) und von dem ersten Ventilelement (24), auch wenn sich das Griffstück (35) in seiner distalen Endposition befindet, in distaler Richtung beabstandet ist und die insbesondere an dem von der Knickschutzhülse (46) lose ummantelten Längenteilabschnitt der Katheterleitung (2) angeordnet ist.

16. Ballonkatheter (1) nach einem oder mehreren der vorangehenden Ansprüche, **dadurch gekennzeichnet, dass** eine Befüllungseinrichtung (56) vorhanden ist, die eine Hohlnadel (57) und ein daran anschließendes Gehäuse (58) umfasst, wobei an der Befüllungseinrichtung (56) zumindest ein Anschluss (6) zur Zufuhr von Fluid zum Befüllen des Ballons (3), insbesondere ein Anschluss (59) zur lösbaren Befestigung einer Spritze (10), ausgebildet ist, dass der Anschluss (59) und die Hohlnadel (57) mittels eines sich in dem Gehäuse (58) erstreckenden Fluiddurchlasses (60) miteinander in fluidischer Verbindung stehen und dass der Außenquerschnitt der Hohlnadel (57) geringfügig größer als der Hohlquerschnitt des ersten Kanals (15) in dem zweiten Kanallängenabschnitt (29) ist.

17. Ballonkatheter (1) nach einem oder mehreren der vorangehenden Ansprüche, **dadurch gekennzeichnet, dass** die Anschlusseinrichtung (6) einen dritten Anschluss (9) aufweist und dass die Katheterleitung (2) einen zweiten Kanal (16) aufweist, der den dritten Anschluss (9) mit einer Öffnung (49) der Katheterleitung (2) an ihrem distalen Ende (5) verbindet.

## Claims

1. Balloon catheter (1) comprising a catheter line (2), an expandable balloon (3) which is attached thereto and borders a balloon interior (4), and a connection device (6) connected to the catheter line (2), which connection device has at least one first connector (7) for supplying fluid, in particular air, for filling the balloon (3), at least one first channel (15) extending in the catheter line (2), which channel is fluidically connected at the proximal longitudinal end (17) thereof to the first connector (7), and is fluidically connected at the distal longitudinal end (26) thereof to the balloon interior (4), the first channel (15) having a first channel length portion (27) and a second channel length portion (29) connected to the first channel length portion in the distal direction by means of a transition (28), the catheter line (2) being made of elastic material, at least along these channel length portions (27, 29) and along the transition (28), the balloon catheter (1) having at least one first valve element (24) which is in particular designed as a sphere and is inserted into the first channel (15), the area of the hollow cross section of the first channel (15) in the second channel length portion (29), in particular when the relevant wall of the catheter line (2) is relaxed, being smaller than the area of the hollow cross section of the first channel (15) in the first channel length portion (27) and smaller than the area of the cross section of the first valve element (24) that is oriented perpendicularly to the longitudinal direction (L) of the catheter line (2), and the balloon catheter (1) having a force transmission device (11), **characterised in that**, the force transmission device (11) is fastened to the first valve element (24) and extends from the first valve element (24) in the proximal direction through the first channel (15) and further through the connection device (6) to a handle piece (35), the first valve element (24) being moveable from the first channel length portion (27) into the second channel length portion (29) by means of a movement of the handle piece (35) towards the connection device (6), with elastic expansion of the wall (30) of the catheter line (2) in said second channel length portion.

2. Balloon catheter (1) according to claim 1, **characterised in that** the area of the hollow cross section of the first channel (15) at each point in the second channel length portion (29) is smaller than the area of the hollow cross section of the first channel (15) at each point in the first channel length portion (27) and is smaller than the area of the largest cross section of the first valve element (24) that is oriented perpendicularly to the longitudinal direction (L) of the catheter line (2).

3. Balloon catheter (1) according to one or more of the preceding claims, **characterised in that**, in particular when the relevant wall (30) of the catheter line (2) is relaxed, the hollow cross section of the first channel (15) in the first channel length portion (27) is uniform or substantially uniform, substantially uniform meaning that any differences in size are only in the single-digit percentage range, and the hollow cross section of the first channel (15) in the second channel length portion (29), which deviates from said hollow cross section of the first channel in the first channel length portion in terms of area, is likewise uniform or substantially uniform in this respect, substantially uniform meaning that any differences in size are only in the single-digit percentage range.

4. Balloon catheter (1) according to claim 1, **characterised in that**, in particular when the wall (30) of the catheter line (2) is relaxed, the area of the hollow cross section of the first channel (15) in the first channel length portion (27) is greater than or equal to or smaller than the area of the cross section of the first valve element (24).

5. Balloon catheter (1) according to one or more of the preceding claims, **characterised in that**, in particular when the relevant wall (30) of the catheter line (2) is relaxed, the first channel (15) in the first channel length portion (27) has a circular hollow cross section having a first channel diameter (dk1) which is uniform in the first channel length portion (27), and in the second channel length portion (29) has a circular hollow cross section having a second channel diameter (dk2) which is uniform in the second channel length portion (29), the second channel diameter (dk2) being smaller than the first channel diameter (dk1) and smaller than the diameter of the first valve element (24) which is designed as a sphere or hemisphere.

6. Balloon catheter (1) according to one or more of the preceding claims, **characterised in that**, in particular when the relevant wall (30) of the catheter line (2) is relaxed, in the first channel length portion (27), the first channel diameter (dk1) is greater than or equal to or smaller than the diameter (dv1) of the first valve element (24) which is designed as a sphere or hemisphere.

7. Balloon catheter (1) according to one or more of the preceding claims, **characterised in that**, in the first channel (15) in the transition (28) between the first channel length portion (27) and the second channel length portion (29), the hollow cross section of the first channel (15) tapers continuously, in particular conically, in the distal direction.

8. Balloon catheter (1) according to one or more of the preceding claims, **characterised in that** a first stop (38) is fastened to or formed on the force transmission device (11), and **in that** a first counter-stop (39) is fastened to or formed on the connection device (6), and **in that** the handle piece (35) can be advanced towards the connection device (6) to a distal end position, the first stop (38) coming up against the first counter-stop (39) so as to limit the movement in a form-fitting manner and the first valve element (24) being located in the second channel length portion (29) when said distal end position is reached.

9. Balloon catheter (1) according to one or more of the preceding claims, **characterised in that** a second stop (43) is fastened to or formed on the force transmission device (11) and a second counter-stop (44) is fastened to or formed on the connection device (6), and **in that** the handle piece (35) can be pulled away from the connection device (6) to a proximal end position, the second stop (43) coming up against the second counter-stop (44) so as to limit the pulling movement in a form-fitting manner, and the first valve element (24) being located in the first channel length portion (27) when said proximal end position is reached.

10. Balloon catheter (1) according to one or more of the preceding claims, **characterised in that** the balloon catheter (1) has a second, in particular cylindrical, valve element (25) which adjoins the first valve element (24) on the proximal face thereof, the diameter (dv2) of the second valve element (25) in particular corresponding to the diameter (dv1) of the first valve element (24), the second valve element (25) being located entirely or partially in the second channel length portion (29) of the first channel (15) when the handle piece (35) is in the distal end position.

11. Balloon catheter (1) according to one or more of the preceding claims, **characterised in that** the proximal longitudinal end of the second channel length portion (29) is spaced apart from the connection device (6) in the distal direction.

12. Balloon catheter (1) according to one or more of the preceding claims, **characterised in that** the outer surface of the catheter line (2), at least along the first and second channel length portions (27, 29) and the transition (28), is made of plastics material, such as PTFE or rubber.

13. Balloon catheter (1) according to one or more of the preceding claims, **characterised in that** the catheter line (2) is formed in one piece, at least in a partial length portion including the first channel length portion (27), the transition (28) and the second channel length portion (29).

14. Balloon catheter (1) according to one or more of the preceding claims, **characterised in that** the balloon catheter (1) has a anti-kink sleeve (46) which is attached to the connection device (6) and which, from there, loosely encases a length portion of the catheter line that extends from the connection device (6) in the distal direction into the second channel length portion (29), and **in that** a first marking (47) which is visible from the outside, in particular a ring in a first colour, is attached to the anti-kink sleeve (46), the first marking (47) being spaced apart from the distal longitudinal end of the first channel length portion (27) in the proximal direction.

15. Balloon catheter (1) according to one or more of the preceding claims, **characterised in that** a second marking (48), in particular a ring in a second colour, is attached to the catheter line (2) which second marking is visible from the outside when the catheter line (2) is exposed, the second marking (48) being spaced apart in the distal direction from the proximal longitudinal end of the second channel length portion (29) and from the first valve element (24), even when the handle piece (35) is in the distal end position thereof, and said marking in particular being arranged on the partial length portion of the catheter line (2) that is loosely encased by the anti-kink sleeve (46).

16. Balloon catheter (1) according to one or more of the preceding claims, **characterised in that** a filling device is provided (56) which comprises a hollow needle (57) and a housing (58) connected thereto, at least one connector (6) for supplying fluid for filling the balloon (3), in particular a connector (59) for detachably fastening a syringe (10), being formed on the filling device (56), that the connector (59) and the hollow needle (57) are fluidically interconnected by means of a fluid passage (60) extending in the housing (58), and **in that** the outer cross section of the hollow needle (57) is slightly larger than the hollow cross section of the first channel (15) in the second channel length portion (29).

17. Balloon catheter (1) according to one or more of the preceding claims, **characterised in that** the connection device (6) has a third connector (9) and **in that** the catheter line (2) has a second channel (16) which connects the third connector (9) to an opening (49) of the catheter line (2) at the distal end (5) thereof.

## Revendications

1. Cathéter à ballonnet (1), comprenant une conduite de cathéter (2), un ballonnet (3) expansible fixé à celle-ci, qui délimite un espace intérieur de ballonnet (4), et un dispositif de raccordement (6) lié à la conduite de cathéter (2), qui présente au moins un premier raccord (7) pour l'alimentation en fluide, en particulier en air, pour le remplissage du ballonnet (3), dans lequel au moins un premier canal (15) s'étend dans la conduite de cathéter (2), qui est en liaison de fluide avec le premier raccord (7) à son extrémité longitudinale proximale (17) et qui est en liaison de fluide avec l'intérieur du ballonnet (4) à son extrémité longitudinale distale (26), dans lequel le premier canal (15) présente un premier tronçon longitudinal de canal (27) et un deuxième tronçon longitudinal de canal (29) qui s'y raccorde dans la direction distale avec une transition (28), dans lequel la conduite de cathéter (2) est réalisée en matériau élastique au moins le long de ces tronçons longitudinaux de canal (27, 29) et le long de la transition (28), dans lequel le cathéter à ballonnet (1) présente au moins un premier élément de valve (24), en particulier conçu sous forme de bille, qui est inséré dans le premier canal (15), dans lequel, en particulier lorsque la paroi de la conduite de cathéter (2) est respectivement détendue, la surface de la section transversale creuse du premier canal (15) dans le deuxième tronçon longitudinal de canal (29) est plus petite que la surface de la section transversale creuse du premier canal (15) dans le premier tronçon longitudinal de canal (27) et plus petite que la surface de la section transversale du premier élément de valve (24) orientée perpendiculairement à la direction longitudinale (L) de la conduite de cathéter (2), et dans lequel le cathéter à ballonnet (1) présente un dispositif de transmission de force (11), **caractérisé en ce que** le dispositif de transmission de force (11) est fixé au premier élément de valve (24) et s'étend depuis le premier élément de valve (24) en direction proximale à travers le premier canal (15) et après à travers les moyens de raccordement (6) jusqu'à une poignée (35), dans lequel le premier élément de valve (24) peut, au moyen d'un mouvement de la poignée (35) par rapport au dispositif de raccordement (6), être déplacé du premier tronçon longitudinal de canal (27) dans le deuxième tronçon longitudinal de canal (29) avec une expansion élastique de la paroi (30) de la conduite de cathéter (2) à son endroit.

2. Cathéter à ballonnet (1) selon la revendication 1, **caractérisé en ce que** la surface de la section transversale creuse du premier canal (15) à chaque endroit dans le deuxième tronçon longitudinal de canal (29) est plus petite que la surface de la section transversale creuse du premier canal (15) à chaque endroit dans le premier tronçon longitudinal de canal (27) et plus petite que la surface de la plus grande section transversale du premier élément de valve (24) orientée perpendiculairement à la direction longitudinale (L) de la conduite de cathéter (2).

3. Cathéter à ballonnet (1) selon une ou plusieurs des revendications précédentes, **caractérisé en ce que**, en particulier avec la paroi (30) de la conduite de cathéter (2) respectivement détendue, la section transversale creuse du premier canal (15) dans le premier tronçon longitudinal de canal (27) est uniforme ou sensiblement uniforme, où sensiblement uniforme signifie que toute différence de taille se situe uniquement dans une fourchette de pourcentage à un chiffre, et la section creuse du premier canal (15) dans le deuxième tronçon longitudinal de canal (29) qui s'en écarte en termes de surface est également uniforme ou sensiblement uniforme dans celui-ci, où sensiblement uniforme signifie que toute différence de taille se situe uniquement dans une fourchette de pourcentage à un chiffre.

4. Cathéter à ballonnet (1) selon la revendication 1, **caractérisé en ce que**, en particulier lorsque la paroi (30) de la ligne de cathéter (2) est détendue, la surface de la section transversale creuse du premier canal (15) dans le premier tronçon longitudinal de canal (27) est supérieure ou égale ou inférieure à la surface de la section transversale du premier élément de soupape (24).

5. Cathéter à ballonnet (1) selon une ou plusieurs des revendications précédentes, **caractérisé en ce que**, en particulier lorsque la paroi (30) de la conduite de cathéter (2) est respectivement détendue, le premier canal (15) présente une section transversale creuse circulaire dans le premier tronçon longitudinal de canal (27) avec un premier diamètre de canal uniforme (dk1) dans le premier tronçon longitudinal de canal (27) et une section transversale creuse circulaire dans le deuxième tronçon longitudinal de canal (29) avec un deuxième diamètre de canal uniforme (dk2) dans le deuxième tronçon longitudinal de canal (29), dans lequel le deuxième diamètre de canal (dk2) est plus petit que le premier diamètre de canal (dk1) et plus petit que le diamètre du premier élément de valve (24) qui est formé comme une sphère ou un hémisphère.

6. Cathéter à ballonnet (1) selon une ou plusieurs des revendications précédentes, **caractérisé en ce que**, en particulier avec la paroi (30) de la conduite de cathéter (2) respectivement détendue, dans le premier tronçon longitudinal de canal (27), le diamètre du premier canal (dk1) est supérieur ou égal ou inférieur au diamètre (dv1) du premier élément de soupape (24) conçu comme une bille ou une demi-bille.

7. Cathéter à ballonnet (1) selon une ou plusieurs des revendications précédentes, **caractérisé en ce que** dans le premier canal (15), dans la transition (28) entre le premier tronçon longitudinal de canal (27) et le deuxième tronçon longitudinal de canal (29), la section transversale creuse du premier canal (15) se rétrécit de manière continue, en particulier de manière conique, dans la direction distale.

8. Cathéter à ballonnet (1) selon une ou plusieurs des revendications précédentes, **caractérisé en ce qu'**une première butée (38) est fixée ou formée sur le dispositif de transmission de force (11) et qu'une première contre-butée (39) est fixée ou formée sur le dispositif de raccordement (6) et que la poignée (35) peut être avancée vers le dispositif de raccordement (6) jusqu'à une position d'extrémité distale où, en atteignant celle-ci, la première butée (38) vient buter contre la première contre-butée (39) de manière à limiter le déplacement par complémentarité de forme et le premier élément de valve (24) est situé dans le deuxième tronçon longitudinal de canal (29).

9. Cathéter à ballonnet (1) selon une ou plusieurs des revendications précédentes, **caractérisé en ce qu'**une deuxième butée (43) est fixée ou formée sur le dispositif de transmission de force (11) et une deuxième contre-butée (44) est fixée ou formée sur le dispositif de raccordement (6), et **en ce que** la poignée (35) peut être retirée du dispositif de raccordement (6) jusqu'à une position finale proximale où, en atteignant celle-ci, la deuxième butée (43)vient buter contre la deuxième contre-butée (44) de manière à limiter le mouvement de traction par complémentarité de forme et le premier élément de valve (24) se trouve dans le premier tronçon longitudinal du canal (27).

10. Cathéter à ballonnet (1) selon une ou plusieurs des revendications précédentes, **caractérisé en ce que** le cathéter à ballonnet (1) présente un deuxième élément de valve (25), en particulier de forme cylindrique, contigu au premier élément de soupape (24) sur son côté proximal, dans lequel, en particulier, il est prévu que le diamètre (dv2) du deuxième élément de valve (25) correspond au diamètre (dv1) du premier élément de valve (24), dans lequel, lorsque la poignée (35) est en position d'extrémité distale, le deuxième élément de valve (25) est situé entièrement ou partiellement dans le deuxième tronçon longitudinal de canal (29) du premier canal (15).

11. Cathéter à ballonnet (1) selon une ou plusieurs des revendications précédentes, **caractérisé en ce que** l'extrémité longitudinale proximale du deuxième tronçon longitudinal de canal (29) est espacée en direction distale du dispositif de raccordement (6).

12. Cathéter à ballonnet (1) selon une ou plusieurs des revendications précédentes, **caractérisé en ce que** la surface extérieure de la conduite de cathéter (2) est en plastique tel que du PTFE ou en caoutchouc au moins le long des premier et deuxième tronçons longitudinaux de canal (27, 29) et de la transition (28).

13. Cathéter à ballonnet (1) selon une ou plusieurs des revendications précédentes, **caractérisé en ce que** la conduite de cathéter (2) est formée en une seule pièce au moins dans une partie longitudinale comprenant le premier tronçon longitudinal de canal (27), la transition (28) et le deuxième tronçon longitudinal de canal (29).

14. Cathéter à ballonnet (1) selon une ou plusieurs des revendications précédentes, **caractérisé en ce que** le cathéter à ballonnet (1) présente un manchon anti-pliage (46) qui est fixé au dispositif de raccordement (6) et qui, à partir de là, entoure de manière lâche une partie longitudinale de la conduite de cathéter qui s'étend du dispositif de raccordement (6) dans la direction distale jusqu'au deuxième tronçon longitudinal de canal (29), et qu'un premier marquage (47) visible de l'extérieur, en particulier un anneau d'une première couleur, est apposé sur le manchon anti-pliage (46), le premier marquage (47) étant espacé de l'extrémité longitudinale distale du premier tronçon longitudinal de canal (27) dans la direction proximale.

15. Cathéter à ballonnet (1) selon une ou plusieurs des revendications précédentes, **caractérisé en ce qu'**un deuxième marquage (48), en particulier un anneau d'une deuxième couleur, est apposé sur la conduite de cathéter (2), lequel marquage est visible de l'extérieur lorsque la conduite de cathéter (2) est exposée, dans lequel le deuxième marquage (48) est espacé dans la direction distale de l'extrémité longitudinale proximale du deuxième tronçon longitudinal de canal (29) et du premier élément de valve (24), même lorsque la poignée (35) est dans sa position d'extrémité distale, et qui est agencé en particulier sur la partie longitudinale de la conduite de cathéter (2) entourée de manière lâche par le manchon anti-pliage (46).

16. Cathéter à ballonnet (1) selon une ou plusieurs des revendications précédentes, **caractérisé en ce qu'**il est prévu un dispositif de remplissage (56) qui comprend une aiguille creuse (57) et un boîtier (58) se raccordant à celle-ci, dans lequel est prévu sur le dispositif de remplissage (56) au moins un raccord (6) pour l'alimentation en fluide pour le remplissage du ballonnet (3), en particulier un raccord (59) pour la fixation amovible d'une seringue (10), **en ce que** le raccord (59) et l'aiguille creuse (57) sont en liaison de fluide l'un avec l'autre au moyen d'un passage de fluide (60) s'étendant dans le boîtier (58) et **en ce que** la section transversale extérieure de l'aiguille creuse (57) est légèrement plus grande que la section transversale creuse du premier canal (15) dans le deuxième tronçon longitudinal de canal (29).

17. Cathéter à ballonnet (1) selon une ou plusieurs des revendications précédentes, **caractérisé en ce que** le dispositif de raccordement (6) comporte un troisième raccord (9) et **en ce que** la conduite de cathéter (2) comporte un deuxième canal (16) reliant le troisième raccord (9) à une ouverture (49) de la conduite de cathéter (2) à son extrémité distale (5).
